# EUROPEAN PATENT APPLICATION

(11) **EP 4 406 968 A1**
(43) Date of publication of application: **31.07.2024**
(21) Application number: 22872115.5
(22) Date of filing: 23.09.2022
(51) Int. Cl.: C07K 16/00, C07K 16/28, A61K 39/00, A61K 39/395

(54) **ANTI-KLB ANTIBODIES AND USES**

(30) Priority: 23.09.2021 CN 202111110895
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: ZHANG, Ling, Shanghai 200245 (CN); YING, Hua, Shanghai 200245 (CN); ZHANG, Mingxi, Shanghai 200245 (CN); JIN, Xinsheng, Shanghai 200245 (CN); TAO, Weikang, Shanghai 200245 (CN)
(74) Representative: Regimbeau
(86) International application number: PCT/CN2022/120887
(87) International publication number: WO 2023/046071

(57) **Abstract**

The present disclosure relates to anti-KLB antibodies and uses.

## Description

### TECHNICAL FIELD

The present disclosure belongs to the field of biotechnology, and specifically, the present disclosure relates to an anti-KLB antibody and use thereof.

### BACKGROUND

The statements herein merely provide background information related to the present disclosure and may not necessarily constitute the prior art.

KLB (Klotho-beta or β-Klotho) is one of the members of the Klotho family of proteins and consists of an extracellular domain, a transmembrane domain, and an intracellular domain. KLB and fibroblast growth factor receptors (FGFRs, including FGFR1-4 and alternative splicing variants thereof, e.g., FGFR1c, FGFR2c, and FGFR3c) form complexes, which serve as common receptors for fibroblast growth factors (FGFs, e.g., FGF19 and FGF21). KLB is highly expressed in liver, adipocytes, and pancreas as a determinant of cellular response to FGF21/FGF19 (Kurosu, H. et al., (2007) J Biol Chem. 282, 26687-95). Fibroblast growth factor receptors contain an intracellular tyrosine kinase domain, which is activated upon binding to a ligand to produce MAPK (Erk1/2), RAF1, AKT1, STAT, etc., activating downstream signaling pathways (Kharitonenkov, A. et al., (2008) BioDrugs. 22: 37-44).

Fibroblast growth factor 21 (FGF21) is one of the members of the fibroblast growth factor family and is highly expressed in the liver and pancreas (Itoh et al., (2004) Trend Genet. 20: 563-69). FGF21 binds to KLB via the C-terminus and FGFR via the N-terminus to form a receptor-ligand complex, thereby activating signaling pathways downstream of FGFR. Studies show that on various target organs (e.g., liver, adipose tissue, pancreas, adrenal gland in CNS, etc.) associated with metabolism, FGF21 exerts the functions of regulating glucose metabolism, lipid metabolism, energy expenditure, etc., by forming complexes with KLB&FGFR1c, KLB&FGFR2c, and KLB&FGFR3c. Transgenic mice overexpressing FGF21 exhibited low insulin, low cholesterol, low triglycerides, and increased insulin sensitivity, and weight gain from high-fat foods is prevented (Kharitonenkov et al., (2005) J Clin Invest. 115: 1627-35). FGF21 analogs (e.g. AKR001, see SEQ ID NO: 47 in WO2010129503A1) have been well confirmed in clinical trials as being able to significantly reduce liver lipid, increase NASH resolution, alleviate fibrosis, etc.

### SUMMARY

The present disclosure constructs an anti-KLB antibody, which comprises a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein
(i) the HCDR1, the HCDR2, and the HCDR3 of the heavy chain variable region comprise amino acid sequences of a HCDR1, a HCDR2, and a HCDR3, respectively, in any one of SEQ ID NOs: 40-48, and the LCDR1, the LCDR2, and the LCDR3 of the light chain variable region comprise amino acid sequences of a LCDR1, a LCDR2, and a LCDR3, respectively, in SEQ ID NO: 34; or, the HCDR1, the HCDR2, and the HCDR3 of the heavy chain variable region comprise amino acid sequences of a HCDR1, a HCDR2, and a HCDR3, respectively, in SEQ ID NO: 2, and the LCDR1, the LCDR2, and the LCDR3 of the light chain variable region comprise amino acid sequences of a LCDR1, a LCDR2, and a LCDR3, respectively, in SEQ ID NO: 3; or
(ii) the HCDR1, the HCDR2, and the HCDR3 of the heavy chain variable region comprise amino acid sequences of a HCDR1, a HCDR2, and a HCDR3, respectively, in SEQ ID NO: 49, and the LCDR1, the LCDR2, and the LCDR3 of the light chain variable region comprise amino acid sequences of a LCDR1, a LCDR2, and a LCDR3, respectively, in any one of SEQ ID NOs: 56-68; or, the HCDR1, the HCDR2, and the HCDR3 of the heavy chain variable region comprise amino acid sequences of a HCDR1, a HCDR2, and a HCDR3, respectively, in SEQ ID NO: 4, and the LCDR1, the LCDR2, and the LCDR3 of the light chain variable region comprise amino acid sequences of a LCDR1, a LCDR2, and a LCDR3, respectively, in SEQ ID NO: 5; or
(iii) the HCDR1, the HCDR2, and the HCDR3 of the heavy chain variable region comprise amino acid sequences of a HCDR1, a HCDR2, and a HCDR3, respectively, in SEQ ID NO: 18, and the LCDR1, the LCDR2, and the LCDR3 of the light chain variable region comprise amino acid sequences of a LCDR1, a LCDR2, and a LCDR3, respectively, in SEQ ID NO: 19.

In some embodiments, i) the HCDR1, the HCDR2, and the HCDR3 of the heavy chain variable region comprise amino acid sequences of a HCDR1, a HCDR2, and a HCDR3, respectively, in SEQ ID NO: 40, and the LCDR1, the LCDR2, and the LCDR3 of the light chain variable region comprise amino acid sequences of a LCDR1, a LCDR2, and a LCDR3, respectively, in SEQ ID NO: 34; or ii) the HCDR1, the HCDR2, and the HCDR3 of the heavy chain variable region comprise amino acid sequences of a HCDR1, a HCDR2, and a HCDR3, respectively, in SEQ ID NO: 49, and the LCDR1, the LCDR2, and the LCDR3 of the light chain variable region comprise amino acid sequences of a LCDR1, a LCDR2, and a LCDR3, respectively, in SEQ ID NO: 57; or iii) the HCDR1, the HCDR2, and the HCDR3 of the heavy chain variable region comprise amino acid sequences of a HCDR1, a HCDR2, and a HCDR3, respectively, in SEQ ID NO: 18, and the LCDR1, the LCDR2, and the LCDR3 of the light chain variable region comprise amino acid sequences of a LCDR1, a LCDR2, and a LCDR3, respectively, in SEQ ID NO: 19. In some embodiments, the HCDR1, the HCDR2, and the HCDR3 of the heavy chain variable region comprise amino acid sequences of a HCDR1, a HCDR2, and a HCDR3, respectively, in SEQ ID NO: 40, and the LCDR1, the LCDR2, and the LCDR3 of the light chain variable region comprise amino acid sequences of a LCDR1, a LCDR2, and a LCDR3, respectively, in SEQ ID NO: 34.

In some embodiments, provided is the anti-KLB antibody according to any one of the above, wherein the HCDR1, the HCDR2, and the HCDR3 of the heavy chain variable region and the LCDR1, the LCDR2, and the LCDR3 of the light chain variable region are defined according to the same numbering scheme selected from the group consisting of Kabat, IMGT, Chothia, AbM, and Contact. In some embodiments, the HCDR1, the HCDR2, and the HCDR3 of the heavy chain variable region and the LCDR1, the LCDR2, and the LCDR3 of the light chain variable region are defined according to the Kabat numbering scheme; in some embodiments, the HCDR1, the HCDR2, and the HCDR3 of the heavy chain variable region and the LCDR1, the LCDR2, and the LCDR3 of the light chain variable region are defined according to the IMGT numbering scheme; in some embodiments, the HCDR1, the HCDR2, and the HCDR3 of the heavy chain variable region and the LCDR1, the LCDR2, and the LCDR3 of the light chain variable region are defined according to the Chothia numbering scheme; in some embodiments, the HCDR1, the HCDR2, and the HCDR3 of the heavy chain variable region and the LCDR1, the LCDR2, and the LCDR3 of the light chain variable region are defined according to the AbM numbering scheme; in some embodiments, the HCDR1, the HCDR2, and the HCDR3 of the heavy chain variable region and the LCDR1, the LCDR2, and the LCDR3 of the light chain variable region are defined according to the Contact numbering scheme.

In some embodiments, provided is the anti-KLB antibody according to any one of the above, wherein:
i) in the heavy chain variable region, the HCDR1 comprises the amino acid sequence of SEQ ID NO: 6, the HCDR2 comprises the amino acid sequence of SEQ ID NO: 37, 38, 39, or 7, and the HCDR3 comprises the amino acid sequence of SEQ ID NO: 8; and in the light chain variable region, the LCDR1 comprises the amino acid sequence of SEQ ID NO: 9, the LCDR2 comprises the amino acid sequence of SEQ ID NO: 10, and the LCDR3 comprises the amino acid sequence of SEQ ID NO: 11; or
ii) in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14; and in the light chain variable region, the LCDR1 comprises the amino acid sequence of SEQ ID NO: 15, 69, 70, 71, 72, 73, or 74, the LCDR2 comprises the amino acid sequence of SEQ ID NO: 16 or 75, and the LCDR3 comprises the amino acid sequence of SEQ ID NO: 17; or
iii) in the heavy chain variable region, the HCDR1 comprises the amino acid sequence of SEQ ID NO: 24, the HCDR2 comprises the amino acid sequence of SEQ ID NO: 25, and the HCDR3 comprises the amino acid sequence of SEQ ID NO: 26; and in the light chain variable region, the LCDR1 comprises the amino acid sequence of SEQ ID NO: 27, the LCDR2 comprises the amino acid sequence of SEQ ID NO: 28, and the LCDR3 comprises the amino acid sequence of SEQ ID NO: 29.

In some embodiments, provided is the anti-KLB antibody according to any one of the above, wherein
i) in the heavy chain variable region, the HCDR1 comprises the amino acid sequence of SEQ ID NO: 6, the HCDR2 comprises the amino acid sequence of SEQ ID NO: 37, and the HCDR3 comprises the amino acid sequence of SEQ ID NO: 8; and in the light chain variable region, the LCDR1 comprises the amino acid sequence of SEQ ID NO: 9, the LCDR2 comprises the amino acid sequence of SEQ ID NO: 10, and the LCDR3 comprises the amino acid sequence of SEQ ID NO: 11; or
ii) in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14; and in the light chain variable region, the LCDR1 comprises the amino acid sequence of SEQ ID NO: 69, the LCDR2 comprises the amino acid sequence of SEQ ID NO: 75, and the LCDR3 comprises the amino acid sequence of SEQ ID NO: 17.

In some embodiments, provided is the anti-KLB antibody according to any one of the above, wherein the HCDR1, the HCDR2, and the HCDR3 of the heavy chain variable region and the LCDR1, the LCDR2, and the LCDR3 of the light chain variable region are defined according to the Kabat numbering scheme.

In some embodiments, provided is the anti-KLB antibody according to any one of the above, wherein the anti-KLB antibody is a murine antibody, a chimeric antibody, a humanized antibody, or a fully human-derived antibody. In some embodiments, the antibody is a human-derived antibody. In some embodiments, the antibody is a fully human antibody.

In some embodiments, provided is the anti-KLB antibody according to any one of the above, wherein: (i) the heavy chain variable region comprises SEQ ID NO: 40, 30, 31, 32, 41, 42, 43, 44, 45, 46, 47, or 48, or an amino acid sequence having at least 90% (e.g., at least 90%, 95%, 96%, 97%, 98%, or 99%) sequence identity thereto, and the light chain variable region comprises SEQ ID NO: 34, 33, 35, or 36, or an amino acid sequence having at least 90% (e.g., at least 90%, 95%, 96%, 97%, 98%, or 99%) sequence identity thereto; or
(ii) the heavy chain variable region comprises SEQ ID NO: 49, 50, or 51, or an amino acid sequence having at least 90% (e.g., at least 90%, 95%, 96%, 97%, 98%, or 99%) sequence identity thereto, and the light chain variable region comprises SEQ ID NO: 57, 52, 53, 54, 55, 56, 58, 59, 60, 61, 62, 63, 64, 65, 66, 67, or 68, or an amino acid sequence having at least 90% (e.g., at least 90%, 95%, 96%, 97%, 98%, or 99%) sequence identity thereto; or
(iii) the heavy chain variable region comprises SEQ ID NO: 18 or an amino acid sequence having at least 90% (e.g., at least 90%, 95%, 96%, 97%, 98%, or 99%) sequence identity thereto, and the light chain variable region comprises SEQ ID NO: 19 or an amino acid sequence having at least 90% (e.g., at least 90%, 95%, 96%, 97%, 98%, or 99%) sequence identity thereto; or
(iv) the heavy chain variable region comprises SEQ ID NO: 2 or an amino acid sequence having at least 90% (e.g., at least 90%, 95%, 96%, 97%, 98%, or 99%) sequence identity thereto, and the light chain variable region comprises SEQ ID NO: 3 or an amino acid sequence having at least 90% (e.g., at least 90%, 95%, 96%, 97%, 98%, or 99%) sequence identity thereto; or
(v) the heavy chain variable region comprises SEQ ID NO: 4 or an amino acid sequence having at least 90% (e.g., at least 90%, 95%, 96%, 97%, 98%, or 99%) sequence identity thereto, and the light chain variable region comprises SEQ ID NO: 5 or an amino acid sequence having at least 90% (e.g., at least 90%, 95%, 96%, 97%, 98%, or 99%) sequence identity thereto.

In some embodiments, provided is the anti-KLB antibody according to any one of the above, wherein in the antibody, the heavy chain variable region comprises, in a framework region thereof, one or more amino acid back mutations selected from the group consisting of positions 1, 24, 44, 71, and 91 (numbering according to Kabat numbering scheme), and the light chain variable region comprises, in a framework region thereof, one or more amino acid back mutations selected from the group consisting of positions 2, 4, 36, 38, 43, 44, and 58 (numbering according to Kabat numbering scheme). In some embodiments, provided is the anti-KLB antibody, wherein in the heavy chain variable region, the HCDR1 comprises the amino acid sequence of SEQ ID NO: 6, the HCDR2 comprises the amino acid sequence of SEQ ID NO: 37, 38, 39, or 7, and the HCDR3 comprises the amino acid sequence of SEQ ID NO: 8; and in the light chain variable region, the LCDR1 comprises the amino acid sequence of SEQ ID NO: 9, the LCDR2 comprises the amino acid sequence of SEQ ID NO: 10, and the LCDR3 comprises the amino acid sequence of SEQ ID NO: 11; in the antibody, the heavy chain variable region comprises, in a framework region thereof, one or more amino acid back mutations selected from the group consisting of 1E, 24T, 44G, 71S, and 91F (numbering according to Kabat numbering scheme), and/or the light chain variable region comprises, in a framework region thereof, one or more amino acid back mutations selected from the group consisting of 2V, 4I, 36F, 38E, 43T, 44N, and 58I (numbering according to Kabat numbering scheme).

In some embodiments, provided is the anti-KLB antibody according to any one of the above, wherein in the antibody, the heavy chain variable region comprises, in a framework region thereof, one or more amino acid mutations selected from the group consisting of positions 1, 24, 48, 67, 69, 71, and 73 (numbering according to Kabat numbering scheme), and the light chain variable region comprises, in a framework region thereof, one or more amino acid back mutations selected from the group consisting of positions 2, 45, 47, 49, 58, and 71 (numbering according to Kabat numbering scheme). In some embodiments, provided is the anti-KLB antibody, wherein in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14; and in the light chain variable region, the LCDR1 comprises the amino acid sequence of SEQ ID NO: 15, 69, 70, 71, 72, 73, or 74, the LCDR2 comprises the amino acid sequence of SEQ ID NO: 16 or 75, and the LCDR3 comprises the amino acid sequence of SEQ ID NO: 17; in the antibody, the heavy chain variable region comprises, in a framework region thereof, one or more amino acid mutations selected from the group consisting of 1E, 24G, 48I, 67A, 69V, 71V, and 73K (numbering according to Kabat numbering scheme), and/or the light chain variable region comprises, in a framework region thereof, one or more amino acid back mutations selected from the group consisting of 2N, 45K, 47W, 49Y, 58V, and 71Y (numbering according to Kabat numbering scheme). The mutation positions are numbered according to Kabat numbering scheme; for example, "2N" indicates that residue at position 2 (corresponding to Kabat numbering scheme) is "N".

In some embodiments, provided is the anti-KLB antibody according to any one of the above, wherein
(i) the heavy chain variable region comprises any one of SEQ ID NO: 40, 30, 31, 32, 41, 42, 43, 44, 45, 46, 47, or 48, or an amino acid sequence having at least 90% (e.g., at least 90%, 95%, 96%, 97%, 98%, or 99%) sequence identity thereto, and the light chain variable region comprises any one of SEQ ID NO: 34, 33, 35, or 36, or an amino acid sequence having at least 90% (e.g., at least 90%, 95%, 96%, 97%, 98%, or 99%) sequence identity thereto; or
(ii) the heavy chain variable region comprises any one of SEQ ID NO: 49, 50, or 51, or an amino acid sequence having at least 90% (e.g., at least 90%, 95%, 96%, 97%, 98%, or 99%) sequence identity thereto, and the light chain variable region comprises any one of SEQ ID NO: 57, 52, 53, 54, 55, 56, 58, 59, 60, 61, 62, 63, 64, 65, 66, 67, or 68, or an amino acid sequence having at least 90% (e.g., at least 90%, 95%, 96%, 97%, 98%, or 99%) sequence identity thereto; or
(iii) the heavy chain variable region comprises SEQ ID NO: 18 or an amino acid sequence having at least 90% (e.g., at least 90%, 95%, 96%, 97%, 98%, or 99%) sequence identity thereto, and the light chain variable region comprises SEQ ID NO: 19 or an amino acid sequence having at least 90% (e.g., at least 90%, 95%, 96%, 97%, 98%, or 99%) sequence identity thereto; or
(iv) the heavy chain variable region comprises SEQ ID NO: 2 or an amino acid sequence having at least 90% (e.g., at least 90%, 95%, 96%, 97%, 98%, or 99%) sequence identity thereto, and the light chain variable region comprises SEQ ID NO: 3 or an amino acid sequence having at least 90% (e.g., at least 90%, 95%, 96%, 97%, 98%, or 99%) sequence identity thereto; or
(v) the heavy chain variable region comprises SEQ ID NO: 4 or an amino acid sequence having at least 90% (e.g., at least 90%, 95%, 96%, 97%, 98%, or 99%) sequence identity thereto, and the light chain variable region comprises SEQ ID NO: 5 or an amino acid sequence having at least 90% (e.g., at least 90%, 95%, 96%, 97%, 98%, or 99%) sequence identity thereto.

In some embodiments, provided is the anti-KLB antibody according to any one of the above, wherein
(i) the heavy chain variable region comprises SEQ ID NO: 40 or an amino acid sequence having at least 90% (e.g., at least 90%, 95%, 96%, 97%, 98%, or 99%) sequence identity thereto, and the light chain variable region comprises SEQ ID NO: 34 or an amino acid sequence having at least 90% (e.g., at least 90%, 95%, 96%, 97%, 98%, or 99%) sequence identity thereto;
(ii) the heavy chain variable region comprises SEQ ID NO: 49 or an amino acid sequence having at least 90% (e.g., at least 90%, 95%, 96%, 97%, 98%, or 99%) sequence identity thereto, and the light chain variable region comprises SEQ ID NO: 57 or an amino acid sequence having at least 90% (e.g., at least 90%, 95%, 96%, 97%, 98%, or 99%) sequence identity thereto; or
(iii) the heavy chain variable region comprises SEQ ID NO: 18 or an amino acid sequence having at least 90% (e.g., at least 90%, 95%, 96%, 97%, 98%, or 99%) sequence identity thereto, and the light chain variable region comprises SEQ ID NO: 19 or an amino acid sequence having at least 90% (e.g., at least 90%, 95%, 96%, 97%, 98%, or 99%) sequence identity thereto.

In some embodiments, provided is the anti-KLB antibody according to any one of the above, wherein the heavy chain variable region comprises the amino acid sequence of SEQ ID NO: 40, and the light chain variable region comprises the amino acid sequence of SEQ ID NO: 34.

In some embodiments, provided is the anti-KLB antibody according to any one of the above, which comprises a heavy chain constant region and a light chain constant region; in some embodiments, the heavy chain constant region is a human IgG heavy chain constant region; in some embodiments, the heavy chain constant region is a human IgG1, IgG2, IgG3 or IgG4 heavy chain constant region; in some embodiments, the light chain constant region is a human λ or κ light chain constant region; in some embodiments, the heavy chain constant region is a human IgG1 heavy chain constant region and the light chain constant region is a human κ light chain constant region; in some embodiments, the Fc region of the heavy chain constant region has one or more amino acid substitutions capable of reducing the binding of the Fc region to an Fc receptor. In some embodiments, the Fc region has L234A and L235A mutations, S228P mutation, and/or YTE mutations (M252Y, S254T, and T256E), and the numbering of the mutations is according to the EU index. In some embodiments, the heavy chain constant region comprises the amino acid sequence of SEQ ID NO: 22, and the light chain constant region comprises the amino acid sequence of SEQ ID NO: 23.

In some embodiments, provided is the anti-KLB antibody according to any one of the above, which comprises a heavy chain and a light chain, wherein
(i) the heavy chain comprises an amino acid sequence having at least 90% (e.g., at least 90%, 95%, 96%, 97%, 98%, or 99%) sequence identity to SEQ ID NO: 78, and the light chain comprises an amino acid sequence having at least 90% (e.g., at least 90%, 95%, 96%, 97%, 98%, or 99%) sequence identity to SEQ ID NO: 79; or
(ii) the heavy chain comprises an amino acid sequence having at least 90% (e.g., at least 90%, 95%, 96%, 97%, 98%, or 99%) sequence identity to SEQ ID NO: 80, and the light chain comprises an amino acid sequence having at least 90% (e.g., at least 90%, 95%, 96%, 97%, 98%, or 99%) sequence identity to SEQ ID NO: 81; or
(iii) the heavy chain comprises an amino acid sequence having at least 90% (e.g., at least 90%, 95%, 96%, 97%, 98%, or 99%) sequence identity to SEQ ID NO: 20, and the light chain comprises an amino acid sequence having at least 90% (e.g., at least 90%, 95%, 96%, 97%, 98%, or 99%) sequence identity to SEQ ID NO: 21.

In some embodiments, provided is the anti-KLB antibody according to any one of the above, which comprises a heavy chain and a light chain, wherein (i) the heavy chain comprises the amino acid sequence of SEQ ID NO: 78, and the light chain comprises the amino acid sequence of SEQ ID NO: 79; (ii) the heavy chain comprises the amino acid sequence of SEQ ID NO: 80, and the light chain comprises the amino acid sequence of SEQ ID NO: 81; or (iii) the heavy chain comprises the amino acid sequence of SEQ ID NO: 20, and the light chain comprises the amino acid sequence of SEQ ID NO: 21.

In some embodiments, provided is the anti-KLB antibody according to any one of the above, which is an antigen-binding fragment; in some embodiments, the antigen-binding fragment is selected from the group consisting of: Fab, F(ab')₂, Fab', Fd, Fv, dsFv, scFv, Fab, and a diabody.

In some embodiments, the present disclosure provides an isolated anti-KLB antibody that competes for binding to human and/or monkey KLB with the anti-KLB antibody according to any one of the above; in some embodiments, the present disclosure provides an isolated anti-KLB antibody that binds to the same epitope as the anti-KLB antibody according to any one of the above.

In some embodiments, provided is the anti-KLB antibody according to any one of the above, wherein the antibody has one or more of the following characteristics:
A. the anti-KLB antibody has activation activity on cells expressing hKLB&hFGFR1c; in some embodiments, the anti-KLB antibody has high activation activity on cells expressing hKLB&hFGFR1c; in some embodiments, the anti-KLB antibody results in an activation fold of greater than 10 for CHOK1 cells expressing hKLB&hFGFR1c, and/or the anti-KLB antibody results in an activation fold of greater than 2 for HEK293T-hKLB&hFGFR1c cells; in some embodiments, the activation fold is the activation fold of the antibody relative to a blank control; in some embodiments, the activation fold is detected according to Test Examples 4 and 5 of the present disclosure;
B. the anti-KLB antibody has weak activation activity on recombinant cells expressing hFGFR1c (hFGFR1c only, not expressing hKLB); in some embodiments, the anti-KLB antibody results in an activation fold of less than 1 for HEK293-hFGFR1c cells; in some embodiments, the activation fold is the activation fold of the antibody relative to a blank control; in some embodiments, the activation fold is detected according to Test Example 6 of the present disclosure;
C. the anti-KLB antibody has weak activation activity on cells expressing hKLB&hFGFR2c, hKLB&hFGFR3c, and hKLB&hFGFR4; in some embodiments, the anti-KLB antibody has weak activation activity on L6-hKLB&hFGFR2c and/or L6-hKLB&hFGFR4 cells; in some embodiments, the anti-KLB antibody results in an activation fold of less than 2 for L6-hKLB&hFGFR2c and/or L6-hKLB&hFGFR4 cells; in some embodiments, the anti-KLB antibody has weak activation activity on L6-hKLB&hFGFR2c, hKLB&hFGFR3c, and/or L6-hKLB&hFGFR4 cells, and in some embodiments, the anti-KLB antibody results in an activation fold of less than 2 for L6-hKLB&hFGFR2c, hKLB&hFGFR3c, and/or L6-hKLB&hFGFR4 cells; in some embodiments, the anti-KLB antibody results in an activation fold of less than 2 for L6-hKLB&hFGFR2c and L6-hKLB&hFGFR4 cells and an activation fold of less than 5 for L6-hKLB&hFGFR3c cells; in some embodiments, the anti-KLB antibody results in an activation fold of less than 1.4 for L6-hKLB&hFGFR2c cells, an activation fold of less than 1.6 for L6-hKLB&hFGFR4 cells, and an activation fold of less than 5 for L6-hKLB&hFGFR3c cells; in some embodiments, the activation fold is the activation fold of the antibody relative to a blank control; in some embodiments, the activation fold is detected according to Test Example 7 of the present disclosure;
D. the anti-KLB antibody is capable of binding to both human KLB and monkey KLB; in some embodiments, the anti-KLB antibody is capable of binding to human and/or monkey KLB with an EC₅₀ value of less than 2.0E-9M, the EC₅₀ value being detected by flow cytometry;
E. the anti-KLB antibody is capable of binding to human KLB with high affinity; in some embodiments, the antibody is capable of binding to human KLB with a KD value of less than 3.0E-9M, the KD value being detected by the Biacore method.

In some embodiments, the anti-KLB antibody has high activation activity on cells expressing hKLB&hFGFR1c and weak activation activity on cells expressing hKLB&hFGFR2c, hKLB&hFGFR3c, and hKLB&hFGFR4; in some embodiments, the anti-KLB antibody results in an activation fold of greater than 10 for CHOK1 cells expressing hKLB&hFGFR1c and an activation fold of less than 2 for L6-hKLB&hFGFR2c and/or L6-hKLB&hFGFR4 cells, and the activation fold is the activation fold of the antibody relative to a blank control.

In another aspect, the present disclosure provides a pharmaceutical composition, which comprises a therapeutically effective amount of the anti-KLB antibody described above and one or more pharmaceutically acceptable carriers, diluents, buffers, or excipients.

In another aspect, the present disclosure provides an isolated nucleic acid encoding the anti-KLB antibody described above.

In another aspect, the present disclosure provides a vector comprising the nucleic acid molecule described above.

In another aspect, the present disclosure provides a host cell comprising the nucleic acid described above.

In another aspect, the present disclosure provides a method for treating a disease, which comprises the step of administering to a subject the anti-KLB antibody or the pharmaceutical composition described above.

In another aspect, the present disclosure further provides use of the anti-KLB antibody or the pharmaceutical composition described above in the preparation of a medicament for treating a disease.

In another aspect, the present disclosure further provides the anti-KLB antibody or the pharmaceutical composition described above for use as a medicament; in some embodiments, the medicament is used for treating a disease.

In some embodiments, the present disclosure provides a method for treating a disease, disorder, or condition associated with human FGF21 and/or human FGF19, which comprises administering to a subject (e.g., a patient) a therapeutically effective amount of the anti-KLB antibody or the pharmaceutical composition described above.

In some embodiments, the disease described above is a disorder associated with FGF21 and/or FGF 19. In some embodiments, the disorder is selected from the group consisting of a metabolic disorder, an endocrine disorder, and a cardiovascular disorder. In some embodiments, the disorder is selected from the group consisting of obesity, diabetes mellitus (type 1 diabetes mellitus and type 2 diabetes mellitus), pancreatitis, dyslipidemia, nonalcoholic fatty liver disease (NAFLD), nonalcoholic steatohepatitis (NASH), insulin resistance, hyperinsulinemia, glucose intolerance, hyperglycemia, metabolic syndrome, acute myocardial infarction, hypertension, atherosclerosis, peripheral arterial disease, stroke, heart failure, coronary heart disease, renal disease, diabetic complications, neurological diseases, and gastroparesis. In some embodiments, the disease is selected from the group consisting of type 2 diabetes mellitus, obesity, dyslipidemia, NASH, cardiovascular diseases, and metabolic syndrome. In some embodiments, the disease is nonalcoholic steatohepatitis.

In another aspect, the present disclosure further provides a method for inducing FGF21 and/or FGF19 signaling, which comprises contacting a cell expressing KLB and FGFR with the anti-KLB antibody described above.

### DETAILED DESCRIPTION

### Terminology

To facilitate the understanding of the present disclosure, some technical and scientific terms are described below. Unless otherwise specifically defined herein, all technical and scientific terms used herein have the same meaning as commonly understood by those of ordinary skill in the art.

As used in the description and in the claims, the singular forms of "a", "an", and "the" include plural referents unless otherwise clearly indicated in the context.

Unless otherwise clearly stated in the context, throughout the description and the claims, the words "comprise", "have", "include", and the like, should be construed in an inclusive sense as opposed to an exclusive or exhaustive sense.

The term "and/or" is intended to include two meanings, "and" and "or". For example, the phrase "A, B, and/or C" is intended to encompass each of the following: A, B, and C; A, B, or C; A or C; A or B; B or C; A and C; A and B; B and C; A (alone); B (alone); and C (alone).

The three-letter and single-letter codes for amino acids used herein are as described in J. Biol. Chem. 243, p3558 (1968).

The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a different structure from amino acids, but function in a manner similar to naturally occurring amino acids.

The term "amino acid mutation" includes amino acid substitutions (also known as amino acid replacements), deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., replacement of one amino acid with another amino acid having different structural and/or chemical properties. Amino acid substitutions include replacement with non-naturally occurring amino acids or with derivatives of the 20 native amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 366W indicates that an amino acid residue at position 366 is W. T366W indicates that an amino acid residue at position 366 is mutated from the original T to W.

The term "antibody" is used in the broadest sense and encompasses a variety of antibody structures, including but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) so long as they exhibit the desired antigen-binding activity. "Natural antibody" refers to a naturally-occurring immunoglobulin molecule. For example, a natural IgG antibody is a heterotetrameric glycoprotein of about 150,000 Daltons composed of two identical light chains and two identical heavy chains linked by a disulfide bond. From the N-terminus to the C-terminus, each heavy chain has one variable region (VH), also known as variable heavy domain or heavy chain variable region, followed by heavy chain constant regions (CHs). Generally, a native IgG heavy chain constant region comprises three constant domains (CH1, CH2, and CH3). Similarly, from the N-terminus to the C-terminus, each light chain has one variable region (VL), also known as variable light domain or light chain variable domain, followed by one constant light domain (light chain constant region, CL). The term "full-length antibody", "intact antibody", and "whole antibody" are used herein interchangeably and refer to an antibody having a substantially similar structure to a native antibody structure or having heavy chains in an Fc region as defined herein. In a natural intact antibody, the light chain comprises light chain variable region VL and constant region CL, wherein the VL is positioned at the amino terminus of the light chain, and the light chain constant region comprises a κ chain and a λ chain; the heavy chain comprises a variable region VH and constant regions (CH1, CH2, and CH3), wherein the VH is positioned at the amino terminus (also known as the N terminus) of the heavy chain, and the constant regions are positioned at the carboxyl terminus (also known as the C terminus), wherein the CH3 is closest to the carboxyl terminus of the polypeptide, and the heavy chain can be of any isotype, including IgG (including subtypes IgG1, IgG2, IgG3, and IgG4), IgA (including subtypes IgA1 and IgA2), IgM, and IgE.

The term "variable region" or "variable domain" refers to a domain in an antibody that binds to an antigen. Herein, the heavy chain variable region (VH) and light chain variable region (VL) each comprise four conserved framework regions (FRs) and three complementarity determining regions (CDRs). The term "complementarity determining region" or "CDR" refers to a region in the variable domain that primarily contributes to antigen binding; "framework" or "FR" refers to variable domain residues other than CDR residues. A VH comprises 3 CDR regions: HCDR1, HCDR2, and HCDR3; a VL comprises 3 CDR regions: LCDR1, LCDR2, and LCDR3. Each VH and VL is composed of three CDRs and four FRs arranged from the amino terminus (also known as N-terminus) to the carboxyl terminus (also known as C-terminus) in the following order: FR1, CDR1, FR2, CDR2, FR3, CDR3, and FR4. Each VH and VL is composed of three CDRs and four FRs arranged from the amino terminus to the carboxyl terminus in the following order: FR1, CDR1, FR2, CDR2, FR3, CDR3, and FR4. For certain antibodies, a single VH or VL may be sufficient to provide antigen binding specificity. The amino acid sequence boundaries of the CDRs can be determined by a variety of well-known schemes, for example, the "Kabat" numbering scheme (see Kabat et al., (1991), "Sequences of Proteins of Immunological Interest", 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains [J]. 2001), and the ImMunoGenTics (IMGT) numbering scheme (Lefranc, M.P. et al., Dev. Comp. Immunol., 27, 55-77 (2003); Front Immunol. 2018 Oct 16; 9: 2278), and the like. The corresponding relationships among the various numbering schemes are well known to those skilled in the art and are exemplary, as shown in Table 1 below.

**Table 1. Relationships among CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

The term "antibody fragment" or "antigen-binding fragment" refers to a molecule different from an intact antibody; it comprises a moiety of an intact antibody that binds to an antigen to which the intact antibody binds. Examples of antibody fragments include, but are not limited to, Fv, Fab, Fab', Fab'-SH, F(ab')₂, single-domain antibodies, single-chain Fab (scFab), diabodies, linear antibodies, single-chain antibodies (scFv), and multispecific antibodies formed from antibody fragments.

The term "Fc region" or "fragment crystallizable region" is used to define the C-terminal region of the heavy chain of an antibody, including native Fc regions and modified Fc regions. In some embodiments, the Fc region comprises two subunits, which may be identical or different. In some embodiments, the Fc region of the human IgG heavy chain is defined as extending from the amino acid residue at position Cys226 or from Pro230 to its carboxyl terminus. Suitable Fc regions used for the antibodies described herein include Fc regions of human IgG1, IgG2 (IgG2A and IgG2B), IgG3, and IgG4. In some embodiments, the boundaries of the Fc region may also be varied, for example, by deleting the C-terminal lysine of the Fc region (residue 447 according to the EU numbering scheme) or deleting the C-terminal glycine and lysine of the Fc region (residues 446 and 447 according to the EU numbering scheme). Unless otherwise stated, the numbering scheme for the Fc region is the EU numbering scheme, also known as the EU index.

The term "chimeric" antibody refers to an antibody in which a portion of the heavy and/or light chain in the antibody is derived from a particular source or species, while the remainder of the heavy and/or light chain is derived from another different source or species.

The term "humanized" antibody refers to an antibody that retains the reactivity of a non-human antibody while having low immunogenicity in humans. For example, this can be achieved by retaining the non-human CDR regions and replacing the remainder of the antibody with its human counterparts (i.e., the constant regions and the framework region portion of the variable regions).

The terms "human antibody", "human-derived antibody", "fully human antibody", and "complete human antibody" are used interchangeably and refer to antibodies in which the variable and constant regions are human sequences. The term encompasses antibodies that are derived from human genes but have, for example, sequences that have been altered to, e.g., reduce possible immunogenicity, increase affinity, eliminate cysteines that may cause undesired folding, or generate glycosylation sites. The term encompasses antibodies recombinantly produced in non-human cells (that may confer glycosylation not characteristic of human cells). The term also encompasses antibodies that have been cultured in transgenic mice comprising some or all of the human immunoglobulin heavy and light chain loci. The meaning of the human antibody specifically excludes humanized antibodies comprising non-human antigen-binding residues.

The term "affinity" refers to the overall strength of a non-covalent interaction between a single binding site of a molecule (e.g., an antibody) and its binding partner (e.g., an antigen). Unless otherwise indicated, as used herein, binding "affinity" refers to an internal binding affinity that reflects a 1:1 interaction between members of a binding pair (e.g., an antibody and an antigen). The affinity of a molecule X for its ligand Y can be generally denoted by the dissociation constant (KD). Affinity can be determined by conventional methods known in the art, including those described herein.

As used herein, the term "k_{assoc}" or "kₐ" refers to the association rate of a particular antibody-antigen interaction, while the term "k_{dis}" or "k_{d}" refers to the dissociation rate of a particular antibody-antigen interaction. The term "KD" refers to the dissociation constant, which is obtained from the ratio of k_{d} to kₐ (i.e., k_{d}/kₐ) and denoted by a molar concentration (M). The KD value of an antibody can be determined using methods well known in the art. For example, surface plasmon resonance is determined using a biosensing system such as a system, or affinity in a solution is determined by solution equilibrium titration (SET). In some embodiments, the KD values are detected by Biacore.

The term "effector function" refers to biological activities that can be attributed to the Fc region of an antibody (either the native sequence Fc region or the amino acid sequence variant Fc region) and vary with the antibody isotype. Examples of effector functions of an antibody include, but are not limited to: C1q binding and complement-dependent cytotoxicity, Fc receptor binding, antibody-dependent cell-mediated cytotoxicity (ADCC), phagocytosis, down-regulation of cell surface receptors (e.g., B cell receptors), and B cell activation.

The term "antibody-dependent cellular cytotoxicity", "antibody-dependent cell-mediated cytotoxicity", or "ADCC" is a mechanism for inducing cell death, which depends on the interaction of antibody-coated target cells with effector cells with lytic activity, such as natural killer cells (NK), monocytes, macrophages, and neutrophils, via an Fcy receptor (FcγR) expressed on the effector cells. For example, NK cells express FcγRIIIa, while monocytes express FcγRI, FcγRII, and FcγRIIIa. The ADCC activity of the antibodies provided herein can be assessed by *in vitro* assays, using cells expressing the antigen as target cells and NK cells as effector cells. Cell lysis is detected based on the release of a label (e.g., radioactive substrate, fluorescent dye, or natural intracellular protein) from the lysed cells.

The term "antibody-dependent cellular phagocytosis" ("ADCP") refers to a mechanism by which antibody-coated target cells are eliminated by internalization of phagocytic cells such as macrophages or dendritic cells.

The term "complement-dependent cytotoxicity" or "CDC" refers to a mechanism for inducing cell death in which the Fc effector domain of a target-binding antibody binds to and activates a complement component C1q, and C1q then activates the complement cascade, resulting in the death of the target cell. Activation of a complement may also result in the deposition of complement components on the surface of target cells that promote CDC by binding to complement receptors on leukocytes (e.g., CR3).

The term "monoclonal antibody" refers to a population of substantially homogeneous antibodies, that is, the amino acid sequences of the antibody molecules comprised in the population are identical, except for a small number of natural mutations that may exist. In contrast, polyclonal antibody formulations generally comprise a plurality of different antibodies having different amino acid sequences in their variable domains, which are generally specific for different epitopes. "Monoclonal" refers to the characteristics of an antibody obtained from a substantially homogeneous population of antibodies, and should not be construed as requiring the production of the antibody by any particular method. In some embodiments, the antibody provided by the present disclosure is a monoclonal antibody.

The term "antigen" refers to a molecule or portion of a molecule that is capable of being bound by a selective binding agent, such as an antigen-binding protein (including, for example, an antibody), and that is otherwise capable of being used in an animal to produce an antibody that is capable of binding to the antigen. An antigen may have one or more epitopes capable of interacting with different antigen-binding proteins (e.g., antibodies).

The term "epitope" refers to an area or region on an antigen that is capable of specifically binding to an antibody or an antigen-binding fragment thereof. Epitopes can be formed from contiguous strings of amino acids (linear epitope) or comprise non-contiguous amino acids (conformational epitope), e.g., coming in spatial proximity due to the folding of the antigen (i.e., by the tertiary folding of a proteinaceous antigen). The difference between the conformational epitope and the linear epitope is that in the presence of denaturing solvents, the binding of the antibody to the conformational epitope is lost. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, for example, but not limited to, alanine scanning, peptide blotting, peptide cleavage analysis, epitope excision, epitope extraction, chemical modification of the antigen (Prot. Sci. 9 (2000) 487-496), and cross-blocking.

The term "capable of specifically binding", "specifically binding", or "binding" means that an antibody (e.g., an anti-KLB antibody) is capable of binding to a certain antigen or an epitope in the antigen with higher affinity than to other antigens or epitopes. Generally, an antibody binds to an antigen or an epitope in the antigen with an equilibrium dissociation constant (KD) of about 1 × 10⁻⁷ M or less (e.g., about 1 × 10⁻⁸ M or less). In some embodiments, the KD for the binding of an antibody to an antigen is 10% or less (e.g., 1%) of the KD for the binding of the antibody to a non-specific antigen (e.g., BSA or casein). KD may be determined using known methods, for example, by a BIACORE^{®} surface plasmon resonance assay. However, an antibody that specifically binds to an antigen or an epitope within the antigen may have cross-reactivity to other related antigens, e.g., to corresponding antigens from other species (homologous), such as humans or monkeys, e.g., *Macaca fascicularis* (cynomolgus, cyno), *Pan troglodytes* (chimpanzee, chimp), or *Callithrix jacchus* (commonmarmoset, marmoset).

The term "nucleic acid" is used interchangeably herein with the term "polynucleotide" and refers to deoxyribonucleotide or ribonucleotide and a polymer thereof in either single-stranded or double-stranded form. The term encompasses nucleic acids comprising known nucleotide analogs or modified backbone residues or linkages, which are synthetic, naturally occurring, and non-naturally occurring, have similar binding properties to the reference nucleic acid, and are metabolized in a manner similar to the reference nucleotide. Examples of such analogs include, but are not limited to, phosphorothioate, phosphoramidate, methylphosphonate, chiral-methylphosphonate, 2-O-methyl ribonucleotide, and peptide-nucleic acid (PNA). "Isolated" nucleic acid refers to a nucleic acid molecule that has been separated from components of its natural environment. The isolated nucleic acid includes a nucleic acid molecule comprised in a cell that generally comprises the nucleic acid molecule, but the nucleic acid molecule is present extrachromosomally or at a chromosomal position different from its natural chromosomal position. An isolated nucleic acid encoding an anti-KLB antibody refers to one or more nucleic acid molecules encoding the anti-KLB antibody, including such one or more nucleic acid molecules in a single vector or separate vectors, and such one or more nucleic acid molecules present at one or more positions in a host cell. Unless otherwise stated, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with mixed bases and/or deoxyinosine residues.

The terms "polypeptide" and "protein" are used interchangeably herein and refer to a polymer of amino acid residues. The terms apply to amino acid polymers in which one or more amino acid residues are artificial chemical mimics of corresponding naturally occurring amino acids, as well as to naturally occurring amino acid polymers and non-naturally occurring amino acid polymers. Unless otherwise stated, a particular polypeptide sequence also implicitly encompasses conservatively modified variants thereof.

The term sequence "identity" refers to the degree (percentage) to which the amino acids/nucleic acids of two sequences are identical at equivalent positions, when the two sequences are optimally aligned, with gaps introduced as necessary to achieve the maximum percent sequence identity, and without considering any conservative substitutions as part of the sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences. The term "fusion" or "linkage" means that components (e.g., VH and VL in Fv) are linked directly or by a covalent bond via one or more linkers.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that can be transformed into a host cell and comprises a nucleic acid sequence that directs and/or controls (along with the host cell) the expression of one or more heterologous coding regions operably linked thereto. Expression constructs may include, but are not limited to, sequences that affect or control transcription and translation and affect RNA splicing of a coding region operably linked thereto in the presence of an intron.

The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progenies derived therefrom, regardless of the number of passages. Progeny may not be completely identical to parent cells in terms of nucleic acid content and may contain mutations. Mutant progenies that have the same function or biological activity as the cells screened or selected from the group consisting of the initially transformed cells are included herein. Host cells include prokaryotic and eukaryotic host cells, wherein eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichiapastoris*, *Pichia finlandica*, *Pichia trehalophila*, *Pichia koclamae*, *Pichia membranaefaciens*, *Pichia minuta* (*Ogataea minuta, Pichia lindneri*), *Pichiaopuntiae*, *Pichia thermotolerans*, *Pichia salictaria*, *Pichia guercuum*, *Pichia pijperi*, *Pichia stiptis*, *Pichia methanolica*, *Pichia*, *Saccharomycescerevisiae*, *Saccharomyces, Hansenula polymorpha, Kluyveromyces, Kluyveromyces lactis, Candida albicans, Aspergillus nidulans, Aspergillus niger, Aspergillus oryzae, Trichoderma reesei*, *Chrysosporium lucknowense*, *Fusarium sp*., *Fusarium gramineum*, *Fusarium venenatum*, *Physcomitrella patens*, and *Neurospora crassa. Pichia*, any *Saccharomyces*, *Hansenula polymorpha*, any *Kluyveromyces*, *Candida albicans*, any *Aspergillus*, *Trichoderma reesei*, *Chrysosporium lucknowense*, any *Fusarium*, *Yarrowia lipolytica*, and *Neurospora crassa.*

As used herein, the expressions "cell", "cell line", and "cell culture" are used interchangeably, and all such designations include their progenies. Thus, the words "transformant" and "transformed cell" include the primary subject cell and cultures derived therefrom, regardless of the passage number. It is also understood that not all progeny have precisely identical DNA contents due to deliberate or inadvertent mutations. Variant progeny that have the same function or biological activity as the original transformed cell from which they were selected are included.

"Optional" or "optionally" means that the event or circumstance subsequently described may, but does not necessarily, occur, and that the description includes instances where the event or circumstance occurs or does not occur.

The term "pharmaceutical composition" refers to a mixture comprising one or more of the anti-KLB antibodies described herein and other chemical components, for example, physiological/pharmaceutically acceptable carriers and excipients.

The term "pharmaceutically acceptable carrier" refers to an ingredient in a pharmaceutical formulation that is different from the active ingredient and is not toxic to the subject. Pharmaceutically acceptable carriers include, but are not limited to, buffers, excipients, stabilizers, or preservatives.

The term "subject" or "individual" includes both human and non-human animals. Non-human animals include all vertebrates (e.g., mammals and non-mammals) such as non-human primates (e.g., cynomolgus monkeys), sheep, dogs, cows, chickens, amphibians, and reptiles. Unless indicated, the terms "patient" and "subject" are used interchangeably herein. As used herein, the term "cynomolgus monkey (cyno)" or "cynomolgus" refers to *Macaca fascicularis.* In certain embodiments, the individual or subject is a human.

"Administrating" or "giving", when applied to animals, humans, experimental subjects, cells, tissue, organs, or biological fluids, refers to contact of an exogenous drug, a therapeutic agent, a diagnostic agent, or a composition with the animals, humans, subjects, cells, tissue, organs, or biological fluids.

The term "sample" refers to a collection of similar fluids, cells or tissues isolated from a subject, as well as fluids, cells or tissues present within a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura; pericardium; peritoneum; abdominal cavity and other body cavities; fluids collected from bronchial lavage; synovial fluids; liquid solutions in contact with a subject or biological source, e.g., cell and organ culture media (including cell or organ conditioned media); lavage fluids; and the like), tissue biopsy samples, fine needle punctures, surgically excised tissues, organ cultures, or cell cultures.

"Treatment" or "treat" (and grammatical variations thereof) refers to clinical intervention in an attempt to alter the natural course of the treated individual, which may be performed either for prophylaxis or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the antibody of the present disclosure is used to delay the development of or slow the progression of a disease. In some embodiments, the anti-KLB antibodies of the present disclosure (including antibodies that bind to human and/or cynomolgus monkey KLB) have the unique property of mimicking the *in vivo* effects of FGF21 and/or FGF19 and inducing FGF21-like signaling and/or FGF19-like signaling; in some embodiments, the antibodies exhibit activity consistent with the native biological function of FGF21. This property makes anti-KLB antibodies useful for the treatment of metabolic diseases associated with FGF21 and/or FGF19, such as type 2 diabetes mellitus, obesity, dyslipidemia, NASH, cardiovascular diseases, or metabolic syndrome, and for the treatment of any disease, disorder, or condition that requires the mimicking or enhancement of the *in vivo* effects of FGF19 and/or FGF21.

"Effective amount" is generally an amount sufficient to reduce the severity and/or frequency of symptoms, eliminate symptoms and/or underlying causes, prevent the appearance of symptoms and/or their underlying causes, and/or ameliorate or improve damage (e.g., lung disease) caused by or associated with a disease state. In some embodiments, the effective amount is a therapeutically effective amount or a prophylactically effective amount. "Therapeutically effective amount" is an amount sufficient to treat a disease state or condition, particularly a state or condition associated with the disease state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health condition of a patient.

### Anti-KLB antibody of the present disclosure

In one aspect, in the present disclosure, an antibody that specifically binds to KLB is obtained by a number of experiments. In some embodiments, the anti-KLB antibody of the present disclosure has a number of advantageous properties, such as binding to antigen with high affinity, human-monkey cross-reactivity, strong activation activity on hKLB&hFGFR1c cells but weak activation activity on hFGFR1c recombinant cells, selectivity for hKLB&hFGFR2c, hKLB&hFGFR3c, and hKLB&hFGFR4 cells, pharmacokinetic properties, and/or druggability.

In some embodiments, the present disclosure provides an antibody that specifically binds to KLB; in some embodiments, the antibody is a full-length antibody or an antigen-binding fragment thereof (e.g., Fv, Fab, Fab', Fab'-SH, F(ab')₂, single-domain antibodies, single-chain Fab (scFab), diabodies, linear antibodies, or single-chain antibodies (scFv)); in some embodiments, the antibody has one or more of the following functional activities:
A. the anti-KLB antibody has activation activity on cells expressing hKLB&hFGFR1c; in some embodiments, the anti-KLB antibody has high activation activity on cells expressing hKLB&hFGFR1c; in some embodiments, the anti-KLB antibody results in an activation fold of greater than 10 for CHOK1 cells expressing hKLB&hFGFR1c, and/or the anti-KLB antibody results in an activation fold of greater than 2 for HEK293T-hKLB&hFGFR1c cells; in some embodiments, the activation fold is the activation fold of the antibody relative to a blank control; in some embodiments, the activation fold is detected according to Test Examples 4 and 5 of the present disclosure;
B. the anti-KLB antibody has weak activation activity on recombinant cells expressing hFGFR1c (hFGFR1c only, not expressing hKLB); in some embodiments, the anti-KLB antibody results in an activation fold of less than 1 for HEK293-hFGFR1c cells; in some embodiments, the activation fold is the activation fold of the antibody relative to a blank control; in some embodiments, the activation fold is detected according to Test Example 6 of the present disclosure;
C. the anti-KLB antibody has weak activation activity on cells expressing hKLB&hFGFR2c, hKLB&hFGFR3c, and hKLB&hFGFR4; in some embodiments, the anti-KLB antibody has weak activation activity on L6-hKLB&hFGFR2c and/or L6-hKLB&hFGFR4 cells; in some embodiments, the anti-KLB antibody results in an activation fold of less than 2 for L6-hKLB&hFGFR2c and/or L6-hKLB&hFGFR4 cells; in some embodiments, the anti-KLB antibody has weak activation activity on L6-hKLB&hFGFR2c, hKLB&hFGFR3c, and/or L6-hKLB&hFGFR4 cells, and in some embodiments, the anti-KLB antibody results in an activation fold of less than 2 for L6-hKLB&hFGFR2c, hKLB&hFGFR3c, and/or L6-hKLB&hFGFR4 cells; in some embodiments, the anti-KLB antibody results in an activation fold of less than 2 for L6-hKLB&hFGFR2c and L6-hKLB&hFGFR4 cells and an activation fold of less than 5 for L6-hKLB&hFGFR3c cells; in some embodiments, the anti-KLB antibody results in an activation fold of less than 1.4 for L6-hKLB&hFGFR2c cells, an activation fold of less than 1.6 for L6-hKLB&hFGFR4 cells, and an activation fold of less than 5 for L6-hKLB&hFGFR3c cells; in some embodiments, the activation fold is the activation fold of the antibody relative to a blank control; in some embodiments, the activation fold is detected according to Test Example 7 of the present disclosure;
D. the anti-KLB antibody is capable of binding to both human KLB and monkey KLB; in some embodiments, the anti-KLB antibody is capable of binding to human and/or monkey KLB with an EC₅₀ value of less than 2.0E-9M, the EC₅₀ value being detected by flow cytometry;
E. the anti-KLB antibody is capable of binding to human KLB with high affinity; in some embodiments, the antibody is capable of binding to human KLB with a KD value of less than 3.0E-9M, the KD value being detected by the Biacore method.

In some embodiments, the anti-KLB antibody has high activation activity on cells expressing hKLB&hFGFR1c and weak activation activity on cells expressing hKLB&hFGFR2c, hKLB&hFGFR3c, and hKLB&hFGFR4; in some embodiments, the anti-KLB antibody results in an activation fold of greater than 10 for CHOK1 cells expressing hKLB&hFGFR1c and an activation fold of less than 2 for L6-hKLB&hFGFR2c and/or L6-hKLB&hFGFR4 cells, and the activation fold is the activation fold of the antibody relative to a blank control.

In some embodiments, provided is the anti-KLB antibody according to any one of the above, which comprises a heavy chain variable region and a light chain variable region, wherein i) sequences of a HCDR1, a HCDR2, and a HCDR3 of the heavy chain variable region are identical to sequences of a HCDR1, a HCDR2, and a HCDR3, respectively, in any one of SEQ ID NOs: 40-48, and sequences of a LCDR1, a LCDR2, and a LCDR3 of the light chain variable region are identical to amino acid sequences of a LCDR1, a LCDR2, and a LCDR3, respectively, in SEQ ID NO: 34; or, sequences of a HCDR1, a HCDR2, and a HCDR3 of the heavy chain variable region are identical to amino acid sequences of a HCDR1, a HCDR2, and a HCDR3, respectively, in SEQ ID NO: 2, and sequences of a LCDR1, a LCDR2, and a LCDR3 of the light chain variable region are identical to amino acid sequences of a LCDR1, a LCDR2, and a LCDR3, respectively, in SEQ ID NO: 3; or
ii) sequences of a HCDR1, a HCDR2, and a HCDR3 of the heavy chain variable region are identical to amino acid sequences of a HCDR1, a HCDR2, and a HCDR3, respectively, in SEQ ID NO: 49, and sequences of a LCDR1, a LCDR2, and a LCDR3 of the light chain variable region are identical to amino acid sequences of a LCDR1, a LCDR2, and a LCDR3, respectively, in any one of SEQ ID NOs: 56-68; or, sequences of a HCDR1, a HCDR2, and a HCDR3 of the heavy chain variable region are identical to amino acid sequences of a HCDR1, a HCDR2, and a HCDR3, respectively, in SEQ ID NO: 4, and sequences of a LCDR1, a LCDR2, and a LCDR3 of the light chain variable region are identical to amino acid sequences of a LCDR1, a LCDR2, and a LCDR3, respectively, in SEQ ID NO: 5; or
iii) sequences of a HCDR1, a HCDR2, and a HCDR3 of the heavy chain variable region are identical to amino acid sequences of a HCDR1, a HCDR2, and a HCDR3, respectively, in SEQ ID NO:18, and sequences of a LCDR1, a LCDR2, and a LCDR3 of the light chain variable region are identical to amino acid sequences of a LCDR1, a LCDR2, and a LCDR3, respectively, in SEQ ID NO: 19.

In some embodiments, provided is the anti-KLB antibody according to any one of the above, wherein the CDRs are defined according to a numbering scheme selected from the group consisting of Kabat, IMGT, Chothia, AbM, and Contact. In some embodiments,
i) in the heavy chain variable region, the HCDR1 is set forth in SEQ ID NO: 6, the HCDR2 is set forth in SEQ ID NO: 37, 38, 39, or 7, and the HCDR3 is set forth in SEQ ID NO: 8; and
   in the light chain variable region, the LCDR1 is set forth in SEQ ID NO: 9, the LCDR2 is set forth in SEQ ID NO: 10, and the LCDR3 is set forth in SEQ ID NO: 11; or
ii) in the heavy chain variable region, the HCDR1 is set forth in SEQ ID NO: 12, the HCDR2 is set forth in SEQ ID NO: 13, and the HCDR3 is set forth in SEQ ID NO: 14; and
   in the light chain variable region, the LCDR1 is set forth in SEQ ID NO: 15, 69, 70, 71, 72, 73, or 74, the LCDR2 is set forth in SEQ ID NO: 16 or 75, and the LCDR3 is set forth in SEQ ID NO: 17; or
iii) in the heavy chain variable region, the HCDR1 is set forth in SEQ ID NO: 24, the HCDR2 is set forth in SEQ ID NO: 25, and the HCDR3 is set forth in SEQ ID NO: 26; and
   in the light chain variable region, the LCDR1 is set forth in SEQ ID NO: 27, the LCDR2 is set forth in SEQ ID NO: 28, and the LCDR3 is set forth in SEQ ID NO: 29.

Preferably,
i) in the heavy chain variable region, the HCDR1 is set forth in SEQ ID NO: 6, the HCDR2 is set forth in SEQ ID NO: 37, and the HCDR3 is set forth in SEQ ID NO: 8; and
   in the light chain variable region, the LCDR1 is set forth in SEQ ID NO: 9, the LCDR2 is set forth in SEQ ID NO: 10, and the LCDR3 is set forth in SEQ ID NO: 11; or
ii) in the heavy chain variable region, the HCDR1 is set forth in SEQ ID NO: 12, the HCDR2 is set forth in SEQ ID NO: 13, and the HCDR3 is set forth in SEQ ID NO: 14; and
   in the light chain variable region, the LCDR1 is set forth in SEQ ID NO: 69, the LCDR2 is set forth in SEQ ID NO: 75, and the LCDR3 is set forth in SEQ ID NO: 17.

In some embodiments, provided is the anti-KLB antibody according to any one of the above, wherein in the antibody, the framework region of the heavy chain variable region is one that comprises, in a heavy chain framework region of IGHV1-3*01/IGHJ6*01, one or more amino acid back mutations selected from the group consisting of positions 1, 24, 44, 71, and 91 (numbering according to Kabat numbering scheme), and the framework region of the light chain variable region is one that comprises, in a light chain framework region of IGKV1-39*01/IGKJ4*01, one or more amino acid back mutations selected from the group consisting of positions 2, 4, 36, 38, 43, 44, and 58 (numbering according to Kabat numbering scheme). In some embodiments, provided is the anti-KLB antibody, wherein in the heavy chain variable region, the HCDR1 comprises the amino acid sequence of SEQ ID NO: 6, the HCDR2 comprises the amino acid sequence of SEQ ID NO: 37, 38, 39, or 7, and the HCDR3 comprises the amino acid sequence of SEQ ID NO: 8; and in the light chain variable region, the LCDR1 comprises the amino acid sequence of SEQ ID NO: 9, the LCDR2 comprises the amino acid sequence of SEQ ID NO: 10, and the LCDR3 comprises the amino acid sequence of SEQ ID NO: 11; in the antibody, the framework region of the heavy chain variable region is one that further comprises, in a heavy chain framework region of IGHV1-3*01/IGHJ6*01, one or more amino acid back mutations selected from the group consisting of 1E, 24T, 44G, 71S, and 91F (numbering according to Kabat numbering scheme), and/or the framework region of the light chain variable region is one that comprises, in a light chain framework region of IGKV1-39*01/IGKJ4*01, one or more amino acid back mutations selected from the group consisting of 2V, 4I, 36F, 38E, 43T, 44N, and 58I (numbering according to Kabat numbering scheme).

In some embodiments, provided is the anti-KLB antibody according to any one of the above, wherein in the antibody, the framework region of the heavy chain variable region is one that comprises, in a heavy chain framework region of IGHV1-3*01/IGHJ6*01, one or more amino acid mutations selected from the group consisting of positions 1, 24, 48, 67, 69, 71, and 73 (numbering according to Kabat numbering scheme), and the framework region of the light chain variable region is one that comprises, in a light chain framework region of IGKV6-21*02/JGKJ2*01 or IGKV3-20*02/JGKJ2*01, one or more amino acid back mutations selected from the group consisting of positions 2, 45, 47, 49, 58, and 71 (numbering according to Kabat numbering scheme). In some embodiments, provided is the anti-KLB antibody, wherein in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14; and in the light chain variable region, the LCDR1 comprises the amino acid sequence of SEQ ID NO: 15, 69, 70, 71, 72, 73, or 74, the LCDR2 comprises the amino acid sequence of SEQ ID NO: 16 or 75, and the LCDR3 comprises the amino acid sequence of SEQ ID NO: 17; in the antibody, the framework region of the heavy chain variable region is one that comprises, in a heavy chain framework region of IGHV1-3*01/IGHJ6*01, one or more amino acid mutations selected from the group consisting of 1E, 24G, 48I, 67A, 69V, 71V, and 73K (numbering according to Kabat numbering scheme), and/or the framework region of the light chain variable region is one that comprises, in a light chain framework region of IGKV6-21*02/JGKJ2*01 or IGKV3-20*02/JGKJ2*01, one or more amino acid back mutations selected from the group consisting of 2N, 45K, 47W, 49Y, 58V, and 71Y (numbering according to Kabat numbering scheme). The mutation positions are numbered according to Kabat numbering scheme; for example, "2N" indicates that residue at position 2 (corresponding to Kabat numbering scheme) is "N".

### Variants of anti-KLB antibody

In certain embodiments, amino acid sequence variants of the anti-KLB antibody provided herein are encompassed. For example, it may be desirable to improve the binding affinity and/or other biological properties of the antibody. Amino acid sequence variants of an antibody can be prepared by introducing appropriate modifications into the nucleotide sequence encoding the antibody, or by peptide synthesis. Such modifications include, for example, deletions from, and/or insertions into and/or substitutions of residues within the amino acid sequence of the anti-KLB antibody. Any combination of deletion, insertion, and substitution can be made to arrive at the final construct, provided that the final construct possesses the desired characteristics, e.g., antigen-binding.

### Variants with substitutions, insertions, and deletions

In certain embodiments, variants of anti-KLB antibody having one or more amino acid substitutions are provided. Sites of interest for substitutional mutagenesis include CDRs and FRs. Conservative substitutions are shown in Table 2 under the heading of "Preferred substitution". More substantial changes are provided in Table 2 under the heading of "exemplary substitution" and as further described below with reference to amino acid side chain classes. Amino acid substitutions can be introduced into an antibody of interest and the products are screened for a desired activity, e.g., retained/improved antigen-binding, reduced immunogenicity, or improved ADCC or CDC.

**Table 2. Amino acid substitutions**

| Original residue | Exemplary substitution | Preferred substitution |
|---|---|---|
| Ala (A) | Val, Leu, Ile | Val |
| Arg (R) | Lys, Gln, Asn | Lys |
| Asn (N) | Gln, His, Asp, Lys, Arg | Gln |
| Asp (D) | Glu, Asn | Glu |
| Cys (C) | Ser, Ala | Ser |
| Gln (Q) | Asn, Glu | Asn |
| Glu (E) | Asp, Gln | Asp |
| Gly (G) | Ala | Ala |
| His (H) | Asn, Gln, Lys, Arg | Arg |
| Ile (I) | Leu; Val; Met; Ala; Phe; norleucine | Leu |
| Leu (L) | Norleucine; Ile; Val; Met; Ala; Phe | Ile |
| Lys (K) | Arg, Gln, Asn | Arg |
| Met (M) | Leu, Phe, Ile | Leu |
| Phe (F) | Trp, Leu, Val, Ile, Ala, Tyr | Tyr |
| Pro (P) | Ala | Ala |
| Ser (S) | Thr | Thr |
| Thr (T) | Ser | Ser |
| Trp (W) | Tyr, Phe | Tyr |
| Tyr (Y) | Trp, Phe, Thr, Ser | Phe |
| Val (V) | Ile; Leu; Met; Phe; Ala; norleucine | Leu |

According to common side-chain properties, amino acids can be grouped as follows:
(1) hydrophobic: norleucine, Met, Ala, Val, Leu, Ile;
(2) neutral, hydrophilic: Cys, Ser, Thr, Asn, Gln;
(3) acidic: Asp, Glu;
(4) basic: His, Lys, Arg;
(5) residues affecting chain orientation: Gly, Pro;
(6) aromatic: Trp, Tyr, Phe.

Non-conservative substitutions will involve substituting a member of one of these classes for a member of another class.

One type of substitutional variant involves substituting one or more CDR residues of a parent antibody (e.g., a humanized or human antibody). Generally, the resulting variant selected for further study will have changes (e.g., improvements) in certain biological properties (e.g., increased affinity, reduced immunogenicity) relative to the parent antibody, and/or will have substantially retained certain biological properties of the parent antibody. An exemplary substitutional variant is an affinity-matured antibody, which can be conveniently produced, for example, using phage display-based affinity maturation techniques such as those described herein. Briefly, one or more CDR residues are mutated and the variant antibodies are displayed on phages and screened for a particular biological activity (e.g., binding affinity). Changes (e.g., substitutions) can be made in CDRs, for example, to improve antibody affinity. Such changes can be made in CDR "hotspots", i.e., residues encoded by codons that undergo mutation at high frequency during the somatic maturation process, and/or antigen-contacting residues, and meanwhile, the resulting variant VH or VL is tested for binding affinity. In some embodiments of affinity maturation, diversity is introduced into the variable genes selected for maturation by any one of a variety of methods (e.g., error-prone PCR, chain shuffling, or oligonucleotide-directed mutagenesis). A secondary library is then created. The library is then screened to identify any antibody variants with the desired affinity. Another method for introducing diversity involves CDR-directed methods in which several CDR residues (e.g., 4-6 residues at a time) are randomized. CDR residues involved in antigen-binding can be specifically identified, for example, using alanine scanning mutagenesis or modeling. Particularly, HCDR3 and LCDR3 are often targeted. In certain embodiments, substitutions, insertions or deletions may occur within one or more CDRs so long as such changes do not substantially reduce the ability of the antibody to bind to the antigen. For example, conservative changes (e.g., conservative substitutions as provided herein) that do not substantially reduce binding affinity may be made in CDRs. Such changes may be, for example, outside of the antigen-contacting residues in CDRs.

A useful method for identifying residues or regions of an antibody that can be used as mutagenesis targets is called "alanine scanning mutagenesis". In this method, a residue or group of target residues (e.g., charged residues such as Arg, Asp, His, Lys and Glu) are identified and replaced with a neutral or negatively charged amino acid (e.g., Ala or polyalanine) to determine whether the interaction of the antibody with the antigen is affected. Further substitutions may be introduced at amino acid locations that show functional sensitivity to the initial substitutions. In addition, a crystal structure of an antigen-antibody complex may be studied to identify contact points between the antibody and antigen. These contact residues and neighboring residues may be targeted or eliminated as substitution candidates. Variants may be screened to determine whether they contain the desired properties.

Amino acid sequence insertions include amino- and/or carboxyl-terminal fusions ranging in length from 1 residue to polypeptides containing 100 or more residues, and intrasequence insertions of single or multiple amino acid residues. Examples of terminal insertions include an antibody with an N-terminal methionyl residue. Other insertional variants of the antibody molecule include the fusion of the N- or C-terminus of the antibody to an enzyme or a polypeptide that increases the serum half-life of the antibody.

### Engineering of Fc region

In one aspect, the Fc region of the anti-KLB antibody of the present disclosure comprises one or more amino acid substitutions that reduce its binding to an Fc receptor, e.g., its binding to an Fcy receptor, and reduce or eliminate effector functions. A natural IgG Fc region, specifically an IgG₁ Fc region or an IgG₄ Fc region, may cause the anti-KLB antibody of the present disclosure to target cells expressing an Fc receptor, rather than cells expressing an antigen. The engineered Fc region of the present disclosure exhibits reduced binding affinity for an Fc receptor and/or reduced effector functions. In some embodiments, the engineered Fc region shows above 50%, 80%, 90%, or 95% reduction in binding affinity for an Fc receptor as compared to the native Fc region. In some embodiments, the Fc receptor is an Fcy receptor. In some embodiments, the Fc receptor is a human Fcγ receptor, e.g., FcyRI, FcyRIIa, FcyRIIB, or FcyRIIIa. In some embodiments, the engineered Fc region also has reduced binding affinity for a complement (e.g., C1q) as compared to the native Fc region. In some embodiments, the engineered Fc region has no reduced binding affinity for neonatal Fc receptor (FcRn) as compared to the native Fc region. In some embodiments, the engineered Fc region has reduced effector functions, which may include, but are not limited to, one or more of the following: reduced complement-dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen presenting cells, reduced binding to NK cells, reduced binding to macrophages, reduced binding to monocytes, reduced binding to polymorphonuclear cells, reduced direct signaling-induced apoptosis, reduced dendritic cell maturation, and reduced T cell priming. For the IgG₁ Fc region, amino acid residue substitutions at positions such as positions 238, 265, 269, 270, 297, 327, and 329 can reduce effector functions. In some embodiments, the Fc region is a human IgG₁ Fc region, and the amino acid residues at positions 234 and 235 are A, as numbered according to the EU index. For the IgG₄ Fc region, amino acid residue substitutions at positions such as position 228 can reduce effector functions.

The anti-KLB antibody may further comprise disulfide bond engineering, such as 354C of the first subunit and 349C of the second subunit.

The anti-KLB antibody may comprise different antigen-binding moieties fused to the two subunits of the Fc region and thus may result in undesired homodimerization. To improve yield and purity, it is thus advantageous to introduce modifications that promote heterodimerization in the Fc region of the anti-KLB antibody of the present disclosure. In some embodiments, the Fc region of the present disclosure comprises engineering according to the knob-into-hole (KIH) technique, which involves introducing a protuberance structure (knob) at the interface of a subunit and a pore structure (hole) at the interface of another subunit. As such, the protuberance structure can be positioned in the pore structure, thereby promoting the formation of heterodimers and inhibiting the production of homodimers. The protuberance structure is constructed by substituting a small amino acid side chain at the interface of a subunit with a larger side chain (e.g., tyrosine or tryptophan). The pore structure is created at the interface of another subunit by substituting a large amino acid side chain with a smaller amino acid side chain (e.g., alanine or threonine). The protuberance and pore structures are prepared by altering the nucleic acid encoding the polypeptide, with optional amino acid substitutions shown in Table 3 below:

**Table 3. Combinations of KIH mutations**

| | | | | | | | | |
|---|---|---|---|---|---|---|---|---|
| First subunit | T366Y | T366W | T394W | F405W | T366W | T366Y | T366W | F405W |
| | | | | | | F405A | F405W | Y407A |
| Second subunit | Y407T | Y407A | F405A | T394S | T366S | T394W | T394W | T366W |
| | | | | | L358A | | | |
| | | | | | | Y407T | Y407A | T394S |
| | | | | | Y407V | | | |

In addition to the knob-into-hole technique, other techniques for modifying the CH3 domain of the heavy chain of multispecific antibodies to achieve heterodimerization are also known in the art, e.g., WO 96/27011, WO 98/050431, EP 1870459, WO 2007/110205, WO 007/147901, WO 2009/089004, WO 2010/129304, WO 2011/90754, WO 2011/143545, WO 2012/058768, WO 2013/157954, and WO 013/096291.

The C-terminus of the Fc region may be an intact C-terminus ending with amino acid residues PGK or a shortened C-terminus, e.g., a shortened C-terminus in which one or two C-terminal amino acid residues have been removed. In a preferred aspect, the C-terminus of the heavy chain is a shortened C-terminus ending with PG. Thus, in some embodiments, a composition of intact antibodies may comprise antibody populations with all K447 residues and/or G446 + K447 residues removed. In some embodiments, a composition of intact antibodies may comprise antibody populations without a K447 residue and/or G446 + K447 residues removed. In some embodiments, a composition of intact antibodies comprises antibody populations having a mixture of antibodies with and without a K447 residue and/or G446 + K447 residues.

### Recombination Method

An anti-KLB antibody (e.g., antibody) can be produced using recombinant methods. For these methods, one or more isolated nucleic acids encoding the anti-KLB antibody are provided.

In one embodiment, the present disclosure provides an isolated nucleic acid encoding the anti-KLB antibody described above. Such nucleic acids may each independently encode any one of the polypeptide chains described above. In another aspect, the present disclosure provides one or more vectors (e.g., expression vectors) comprising such nucleic acids. In another aspect, the present disclosure provides a host cell comprising such nucleic acids. In one embodiment, provided is a method for preparing an anti-KLB antibody, wherein the method comprises culturing a host cell comprising a nucleic acid encoding the anti-KLB antibody, as provided above, under conditions suitable for expression, and optionally collecting the anti-KLB antibody from the host cell (or host cell medium).

For recombinant production of the anti-KLB antibody, the nucleic acid encoding the protein is isolated and inserted into one or more vectors for further cloning and/or expression in a host cell. Such nucleic acids can be readily isolated and sequenced using conventional procedures, or produced by recombination methods or obtained by chemical synthesis.

Suitable host cells for cloning or expressing a vector encoding the anti-KLB antibody include prokaryotic or eukaryotic cells described herein. For example, the polypeptide or antigen-binding molecule can be produced in bacteria, particularly when glycosylation and Fc effector functions are not required. After expression, the polypeptide or antigen-binding molecule can be isolated from the bacterial cell paste in a soluble fraction and can be further purified.

In addition to prokaryotes, eukaryotic microorganisms such as filamentous fungi or yeast are suitable cloning or expression hosts for vectors encoding anti-KLB antibodies, including fungal and yeast strains. Suitable host cells for expression of anti-KLB antibodies may also be derived from multicellular organisms (invertebrates and vertebrates); examples of invertebrate cells include plant and insect cells. A number of baculovirus strains have been identified, which can be used in combination with insect cells, particularly for transfection of *Spodoptera frugiperda* cells; plant cell cultures may also be used as hosts, see, e.g., US 5959177, US 6040498, US 6420548, US 7125978, and US 6417429; vertebrate cells can also be used as hosts, e.g., mammalian cell lines adapted for growth in a suspension. Other examples of suitable mammalian host cell lines include a SV40-transformed monkey kidney CV1 line (COS-7); a human embryonic kidney line (293 or 293T cells); baby hamster kidney cells (BHK); mouse sertoli cells (TM4 cells); monkey kidney cells (CV1); African green monkey kidney cells (VERO-76); human cervical cancer cells (HELA); canine kidney cells (MDCK); buffalo rat liver cells (BRL3A); human lung cells (W138); human liver cells (Hep G2); mouse mammary tumor cells (MMT 060562); TRI cells; MRCS cells; and FS4 cells. Other suitable mammalian host cell lines include Chinese hamster ovary (CHO) cells, including DHFR-CHO cells; and myeloma cell lines such as Y0, NS0, and Sp2/0. For reviews of certain mammalian host cell lines suitable for the production of the antibody, see, e.g., Yazaki, P. and Wu, A.M., Methods in Molecular Biology, Vol. 248, Lo, B.K.C. (eds.), Humana Press, Totowa, NJ (2004), pp. 255-268.

### Detection and diagnosis

The anti-KLB antibody provided herein can be identified, screened, or characterized for its physical/chemical characteristics and/or biological activities by a variety of assays known in the art. In one aspect, the anti-KLB antibody of the present disclosure is tested for activity, for example, by known methods such as ELISA and western blot.

In certain embodiments, the anti-KLB antibody provided in the present disclosure can be used to detect the presence of KLB in a biological sample. As used herein, the term "detection" encompasses quantitative or qualitative detection. In certain embodiments, the biological sample includes a cell or tissue, such as tumor tissue.

In one embodiment, an anti-KLB antibody for use in a diagnostic or detection method is provided. In one aspect, a method for detecting the presence of KLB in a biological sample is provided. In certain embodiments, the method comprises contacting the biological sample with an anti-KLB antibody under suitable conditions and detecting whether a complex is formed between the detection agent and the antigen. Such methods may be *in vitro* or *in vivo* methods. In one embodiment, an anti-KLB antibody is used to select a subject suitable for treatment; for example, KLB is a biomarker for selecting a patient.

Exemplary disorders that can be diagnosed using the anti-KLB antibody of the present disclosure are, e.g., diabetes mellitus, cardiovascular diseases, insulin resistance, hypertension, thromboembolic disease, or dyslipidemia, especially nonalcoholic steatohepatitis and diabetes mellitus.

In certain embodiments, a labeled anti-KLB antibody is provided. Labels include, but are not limited to, labels or modules that are detected directly (such as fluorescent, chromophoric, electron-dense, chemiluminescent, and radioactive labels), and modules that are detected indirectly (e.g., modules that are detected indirectly through an enzymatic reaction or molecular interaction, such as enzymes or ligands).

### Treatment Method and Route of Administration

Any of the anti-KLB antibodies (e.g., antibodies) provided herein can be used in the treatment method. In yet another aspect, the present disclosure provides use of the anti-KLB antibody in the manufacture or preparation of a medicament. In some embodiments, the disease is a disease or disorder associated with KLB. In some embodiments, the disease is selected from the group consisting of diabetes mellitus, cardiovascular diseases, insulin resistance, hypertension, thromboembolic disease, and dyslipidemia, especially nonalcoholic steatohepatitis and diabetes mellitus. In one such embodiment, the use further comprises administering to a subject an effective amount of at least one additional therapeutic agent (e.g., one, two, three, four, five, or six additional therapeutic agents). The "subject" according to any of the above embodiments may be a human. In some embodiments, the anti-KLB antibodies of the present disclosure (including antibodies that bind to human and/or cynomolgus monkey KLB) have the unique property of mimicking the *in vivo* effects of FGF21 and/or FGF19 and inducing FGF21-like signaling and/or FGF19-like signaling; in some embodiments, the antibodies exhibit activity consistent with the native biological function of FGF21. This property makes the anti-KLB antibodies of the present disclosure useful for the treatment of metabolic diseases associated with FGF21 and/or FGF19, such as type 2 diabetes mellitus, obesity, dyslipidemia, NASH, cardiovascular diseases, or metabolic syndrome, and for the treatment of any disease, disorder, or condition that requires the mimicking or enhancement of the *in vivo* effects of FGF19 and/or FGF21.

In yet another aspect, provided is a pharmaceutical composition comprising the anti-KLB antibody, e.g., for any of the above pharmaceutical uses or treatment methods. In one embodiment, the pharmaceutical composition comprises any of the anti-KLB antibodies provided herein and a pharmaceutically acceptable carrier. In another embodiment, the pharmaceutical composition further comprises at least one additional therapeutic agent.

The anti-KLB antibody of the present disclosure can be used alone or in combination with other agents for treatment. For example, the antibody of the present disclosure can be co-administered with at least one additional therapeutic agent.

The anti-KLB antibody of the present disclosure (and any additional therapeutic agents) may be administered by any suitable means, including parenteral, intrapulmonary, and intranasal administration, and if required for local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection, depending in part on whether the administration is short-term or long-term. Various administration schedules are contemplated herein, including but not limited to, single administration or multiple administrations at multiple time points, bolus administration, and pulse infusion.

The anti-KLB antibody of the present disclosure will be formulated, administered, and applied in a manner consistent with Good Medical Practice. Factors considered in this context include the specific condition being treated, the specific mammal being treated, the clinical state of the individual patient, the cause of the disorder, the site of delivery of the agent, the method of administration, the timing of administration, and other factors known to medical practitioners. The anti-KLB antibody may be formulated with or without one or more agents currently used to prevent or treat the disorder. The effective amount of such additional agents depends on the amount present in the pharmaceutical composition, the type of disorder or treatment, and other factors. These are generally used in the same dosages and routes of administration as described herein, or in about 1% to 99% of the dosages described herein, or in other dosages, and by any route empirically/clinically determined to be appropriate.

For the prevention or treatment of disease, the appropriate dosage of the anti-KLB antibody of the present disclosure (when used alone or in combination with one or more other additional therapeutic agents) will depend on the type of the disease to be treated, the type of the therapeutic molecule, the severity and course of the disease, whether the therapeutic molecule is administered for preventive or therapeutic purposes, previous therapy, the patient's clinical history and response to the therapeutic molecule, and the discretion of the attending physician. The therapeutic molecule is suitably administered to a patient in one or a series of treatments. For example, the daily dose may be between about 1 µg/kg and 100 mg/kg, depending on the factors mentioned above.

### Article of Manufacture

In another aspect of the present disclosure, provided is an article of manufacture, which comprises materials useful for the treatment, prevention, and/or diagnosis of the above disorders. The article of manufacture comprises a container and a label or package insert on or associated with the container. Suitable containers include, for example, bottles, vials, syringes, IV solution bags, and the like. The container may be formed from a variety of materials such as glass or plastic. The container contains a composition effective in the treatment, prevention, and/or diagnosis of a disease, either alone or in combination with another composition, and may have a sterile access port (e.g., the container may be an intravenous solution bag or vial with a stopper pierceable by a hypodermic injection needle). At least one active agent in the composition is the anti-KLB antibody of the present disclosure. The label or package insert indicates that the composition is used to treat the selected condition. Further, the article of manufacture may comprise: (a) a first container containing a composition, wherein the composition comprises the anti-KLB antibody of the present disclosure; and (b) a second container containing a composition, wherein the composition comprises other cytotoxic agents or additional therapeutic agents. The article of manufacture in this embodiment of the present disclosure may further comprise a package insert indicating that the composition may be used to treat a particular condition. Alternatively or additionally, the article of manufacture may further comprise a second (or third) container containing a pharmaceutically acceptable buffer. From a commercial and user standpoint, it may further contain other materials as required, including other buffers, diluents, filters, needles, and syringes.

### Examples and Test Examples

The present disclosure is further described below with reference to the following examples and test examples, which, however, do not limit the present disclosure. The experimental methods in the examples and test examples of the present disclosure in which specific conditions are not specified are generally performed under conventional conditions such as Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated are commercially available conventional reagents.

### Example 1: Construction of Recombinant Cell Lines

Cell strains expressing human KLB&FGFR1c complex (see Uniprot ID: Q86Z14 for human KLB sequence and see Uniprot ID: P11362 for human FGFR1c sequence), cynomolgus monkey KLB&FGFR1c complex (see Genebank ID: EHH53620.1 for cynomolgus monkey KLB sequence and see SEQ ID NO: 1 for cynomolgus monkey FGFR1c sequence), mouse KLB&FGFR1c complex (see Uniprot Q99N32 for mouse KLB sequence and see Uniprot ID: P16092 for mouse FGFR1c sequence), and human FGFR1c alone were constructed.

Method: full-length genes of human KLB, monkey KLB, mouse KLB, human FGFR1c, monkey FGFR1c, and mouse FGFR1c were each cloned into a lentivirus expression vector pCDH (System bioscience, 01. SBI. CD514B-1), wherein the virus expression vector of KLB and the virus expression vector of FGFR1c carried different resistance genes and packaged KLB and FGFR1c viruses of different species, and three plasmids, i.e., pVSV-G, pCMV-dR8.91, and pCDH-KLB or pCDH-FGFR1c, were co-transfected into HEK293T cells (ATCC^{®} CRL-11268) for virus packaging. For constructing cell lines overexpressing the complexes, CHOK1 (ATCC, CCL-61) or HEK293 (ATCC, CRL-1573) cell line was infected with packaged viruses containing KLB and FGFR1c genes at a certain ratio. For constructing the cell line overexpressing human FGFR1c, the CHOK1 or HEK293 cell line was infected with only the virus containing FGFR1c genes. After 48 hours of infection, the virus-containing culture supernatant was removed, and after 5 days of pressure screening with the corresponding antibiotics, CHOK1 or HEK293 monoclonal cells highly expressing KLB&FGFR1c complex or FGFR1c were obtained by flow cytometry sorting. Finally, recombinant cells, such as CHOK1-hKLB&hFGFR1c, CHOK1-mKLB&mFGFR1c, CHOK1-hFGFR1c, CHOK1-hKLB, HEK293-hKLB, HEK293T-hKLB&hFGFR1c, etc., were obtained.

Amino acid sequence of cynomolgus monkey FGFR1c:

MWSWKCLLFWAVLVTATLCTARPSPTLPEQDALPSSEDDDDDDDSSSEEKETDN
TKPNRMPVAPYWTSPEKMEKKLHAVPAAKTVKFKCPSSGTPNPTLRWLKNGKE
FKPDHRIGGYKVRYATWSIIMDSVVPSDKGNYTCIVENEYGSINHTYQLDVVER
SPHRPILQAGLPANKTVALGSNVEFMCKVYSDPQPHIQWLKHIEVNGSKIGPDN
LPYVQILKTAGVNTTDKEMEVLHLRNVSFEDAGEYTCLAGNSIGLSHHSAWLT
VLEALEERPAVMTSPLYLEIIIYCTGAFLISCMVGSVIVYKMKSGTKKSDFHSQM
AVHKLAKSIPLRRQVTVSADSSASMNSGVLLVRPSRLSSSGTPMLAGVSEYELPE
DPRWELPRDRLVLGKPLGEGCFGQVVLAEAIGLDKDKPNRVTKVAVKMLKSDA
TEKDLSDLISEMEMMKMIGKHKNIINLLGACTQDGPLYVIVEYASKGNLREYLQ
ARRPPGLEYCYNPSHNPEEQLSSKDLVSCAYQVARGMEYLASKKCIHRDLAAR
NVLVTEDNVMKIADFGLARDIHHIDYYKKTTNGRLPVKWMAPEALFDRIYTHQ
SDVWSFGVLLWEIFTLGGSPYPGVPVEELFKLLKEGHRMDKPSNCTNELYMMM
RDCWHAVPSQRPTFKQLVEDLDRIVALTSNQEYLDLSMPLDQYSPSFPDTRSSTC
SSGEDSVFSHEPLPEEPCLPRHPAQLANGGLKRR
SEQ ID NO: 1

Note: in the sequence above, the signal peptide (single underlined part), the extracellular region (non-underlined part), the transmembrane region (double underlined part), and the cytoplasmic region (dotted underlined part) are sequentially present Amino acid sequence of human FGF21:

HHHHHHHPIPDSSPLLQFGGQVRQRYLYTDDAQQTEAHLEIREDGTVGGAADQ
SPESLLQLKALKPGVIQILGVKTSRFLCQRPDGALYGSLHFDPEACSFRELLLED

GYNVYQSEAHGLPLHLPGNKSPHRDPAPRGPARFLPLPGLPPALPEPPGILAPQPP
DVGSSDPLSMVGPSQGRSPSYAS
SEQ ID NO: 76

Amino acid sequence of human FGF19:

HHHHHHLAFSDAGPHVHYGWGDPIRLRHLYTSGPHGLSSCFLRIRADGVVDCA
RGQSAHSLLEIKAVALRTVAIKGVHSVRYLCMGADGKMQGLLQYSEEDCAFEE
EIRPDGYNVYRSEKHRLPVSLSSAKQRQLYKNRGFLPLSHFLPMLPMVPEEPED
LRGHLESDMFSSPLETDSMDPFGLVTGLEAVRSPSFEK
SEQ ID NO: 77

### Example 2: Preparation of Anti-Human β-Klotho (KLB) Hybridoma Monoclonal Antibodies

### 1. Mouse immunization and hybridoma fusion

The recombinant cell line CHOK1-hKLB&hFGFR1c expressing human KLB&FGFR1c complex and the recombinant cell line CHOK1-mKLB&mFGFR1c overexpressing mouse KLB&FGFR1c complex constructed in Example 1 of the present disclosure were mixed for immunizing SJL or Balb/C mice or used for cross-immunization of the mice, the immunization was performed by intraperitoneal injection, and the mice were immunized for multiple times until the titer reached a desired value; then the mice with high antibody titer in serum were selected for spleen cell fusion, and the clones that specifically bound to CHOK1-hKLB&hFGFR1c recombinant cell line but did not bind to the human FGFR1c recombinant cell line CHOK1-hFGFR1c were picked out. The hybridoma cell strains mAb-99 and mAb-18 with good activity were obtained by screening and subjected to sequence amplification. Hybridoma cells in the logarithmic growth phase were harvested, and RNA was extracted with Trizol (Invitrogen, Cat No. 15596-018) by following the procedures in the kit instructions and reverse-transcribed using a PrimeScript^{™} reverse transcription kit (Takara, Cat No. 2680A). The cDNA obtained by reverse transcription was subjected to PCR amplification using a mouse Ig-Primer Set (Novagen, TB326 Rev.B 0503) and then sent for sequencing by a sequencing company, and the obtained variable region sequences of the hybridoma clones, murine antibodies mAb-99 and mAb-18, were as follows:
> Amino acid sequence of heavy chain variable region of mAb-99 
EVQLQQSGAELVRPGSSVKMSCKTSGYTFTNYGINWVKQRPRQGLEWIGYIYIG
NGDIEYNAKFKGKATLTSDTSSSTAYMQLSSLTSEDSAIYFCARGRGLGHFDVW
GTGTTVTVSS
SEQ ID NO: 2
> Amino acid sequence of light chain variable region of mAb-99 
DVQITQSPSYLAASPGETITINCRASKSISKFLAWFQEKPGKTNKLLIYSGSTLQS
GIPSRFSGSGSGTDFTLTISGLEPEDFAMYYCQQHNEYPWTFGGGTKLEIK
SEQ ID NO: 3
> Amino acid sequence of heavy chain variable region of mAb-18 QVQLQQSGPELVRPGVSVKISCKGSGYTFTDYAMHWVKQTHAKSLEWIGVIST 
YSGTTNYNQNFKGKATVTVDKSSSTAYMELARLTSEDSAIYYCARSGPLDYWG
QGTSLTVSS
SEQ ID NO: 4
> Amino acid sequence of light chain variable region of mAb-18

ENVLTQSPAIMSASPGEKVTMTCRASSSVSSSYLHWYQQKSGASPKLWIYSTSN
LASGVPERFSGSGSGTSYSLTISSVEAEDAATYYCQQYSGYPLTFGSGTKLEIK
SEQ ID NO: 5

The CDR sequences of the murine antibodies mAb-99 and mAb-18 are shown in Table 4:

**Table 4. CDR sequences of mAb-99 and mAb-18 antibodies**

| Antibody No. | Heavy chain | | Light chain | |
|---|---|---|---|---|
| mAb-99 | HCDR1 | NYGIN (SEQ ID NO: 6) | LCDR1 | RASKSISKFLA (SEQ ID NO: 9) |
| | HCDR2 | YIYIGNGDIEYNAKFKG (SEQ ID NO: 7) | LCDR2 | SGSTLQS (SEQ ID NO: 10) |
| | HCDR3 | GRGLGHFDV (SEQ ID NO: 8) | LCDR3 | QQHNEYPWT (SEQ ID NO: 11) |
| mAb-18 | HCDR1 | DYAMH (SEQ ID NO: 12) | LCDR1 | RASSSVSSSYLH (SEQ ID NO: 15) |
| | HCDR2 | VISTYSGTTNYNQNFKG (SEQ ID NO: 13) | LCDR2 | STSNLAS (SEQ ID NO: 16) |
| | HCDR3 | SGPLDY (SEQ ID NO: 14) | LCDR3 | QQYSGYPLT (SEQ ID NO: 17) |

| | | | | |
|---|---|---|---|---|
| Note: the amino acid residues of the CDRs in the table were identified using the Kabat numbering scheme and annotated. | | | | |

### 2. Screening of human natural phage library

B cells were isolated from human PBMCs, spleen, and lymph node tissues, RNA was extracted, and a natural single-chain phage antibody library was constructed. After the constructed natural single-chain phage antibody library was packaged to form phage particles, panning was performed using recombinant cell lines overexpressing human KLB, and cross-panning was performed using CHOK1-hKLB (see Example 1) and HEK293T-hKLB (see Example 1) recombinant cells. After 3 rounds of panning, phages containing the single-chain antibody were selected from monoclonal colonies, and the binding activity of the phages for human KLB protein was tested by ELISA. Positive clones were picked out, and their light and heavy chain variable regions were fused with the antibody heavy/light chain constant regions (heavy chain constant region set forth in SEQ ID NO: 22, and light chain constant region set forth in SEQ ID NO: 23), respectively, to construct IgG antibodies. The binding activity of the antibodies for CHOK1-hKLB&FGFR1c, CHOK1-cynoKLB&FGFR1c, and CHOK1-hFGFR1c was detected, and finally a fully human antibody hAb-23 with good biological activity was obtained. The specific sequences are as follows:
> Amino acid sequence of heavy chain variable region of hAb-23 
QVQLVQSGAEVKKPGSSVKVSCKASGGTFSSYAISWVRQAPGQGLEWMGGIIPI
FGTANYAQKFQGRVTITADESTSTAYMELSSLRSEDTAVYYCARELVSYYYGMD
VWGQGTMVTVSS
SEQ ID NO: 18
> Amino acid sequence of light chain variable region of hAb-23 
DIVMTQSPSSLSASVGDRVTITCQASQDISNYLNWYQQKPGKAPKLLIYDASNL
ETGVPSRFSGSGSGTDFTFTISSLQPEDIATYYCQQYDNLPLTFGGGTKVEIK
SEQ ID NO: 19
> Amino acid sequence of heavy chain of hAb-23 
QVQLVQSGAEVKKPGSSVKVSCKASGGTFSSYAISWVRQAPGQGLEWMGGIIPI
FGTANYAQKFQGRVTITADESTSTAYMELSSLRSEDTAVYYCARELVSYYYGMD
VWGQGTMVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPEAAGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK
SEQ ID NO: 20
> Amino acid sequence of light chain of hAb-23 
DIVMTQSPSSLSASVGDRVTITCQASQDISNYLNWYQQKPGKAPKLLIYDASNL
ETGVPSRFSGSGSGTDFTFTISSLQPEDIATYYCQQYDNLPLTFGGGTKVEIKRTV
AAPSVFIFPPSDEQLKSGTASVVCLLNNFYPREAKVQWKVDNALQSGNSQESVT
EQDSKDSTYSLSSTLTLSKADYEKHKVYACEVTHQGLSSPVTKSFNRGEC
SEQ ID NO: 21
> Amino acid sequence of human IgG1 heavy chain constant region (containing mutations L234A and L235A) 
ASTKGPSVFPLAPSSKSTSGGTAALGCLVKDYFPEPVTVSWNSGALTSGVHTFPA
VLQSSGLYSLSSVVTVPSSSLGTQTYICNVNHKPSNTKVDKKVEPKSCDKTHTC
PPCPAPEAAGGPSVFLFPPKPKDTLMISRTPEVTCVVVDVSHEDPEVKFNWYVD
GVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPE
NNYKTTPPVLDSDGSFFLYSKLTVDKSRWQQGNVFSCSVMHEALHNHYTQKSL
SLSPGK
SEQ ID NO: 22
> Amino acid sequence of human κ light chain constant region



SEQ ID NO: 23

The CDRs of the human-derived antibody hAb-23 are shown in Table 5:

**Table 5. CDR sequences of hAb-23 antibody**

| Antibody No. | Heavy chain | | Light chain | |
|---|---|---|---|---|
| hAb-23 | HCDR1 | SYAIS (SEQ ID NO: 24) | LCDR1 | QASQDISNYLN (SEQ ID NO: 27) |
| | HCDR2 | GIIPIFGTANYAQKFQG (SEQ ID NO: 25) | LCDR2 | DASNLET (SEQ ID NO: 28) |
| | HCDR3 | ELVSYYYGMDV (SEQ ID NO: 26) | LCDR3 | QQYDNLPLT (SEQ ID NO: 29) |

| | | | | |
|---|---|---|---|---|
| Note: the amino acid residues of the antibody CDRs in the table were identified using the Kabat numbering scheme and annotated. | | | | |

### Example 3: Humanization of Anti-Human KLB Murine Antibodies

By comparing the human antibody heavy and light chain variable region germline gene database with the MOE software, heavy and light chain variable region germline genes with high homology were selected as templates, and CDRs of the murine antibody were grafted into corresponding humanized templates to form variable region sequences in the following order: FR1-CDR1-FR2-CDR2-FR3-CDR3-FR4, and then the variable region sequences were fused with human constant region sequences to obtain the humanized antibody. Humanization of murine antibodies mAb-99 and mAb-18 is described below exemplarily:

### 1. Humanization of mAb-99

IGHV1-3*01/IGHJ6*01 was selected as the template of the antibody mAb-99 humanized heavy chain, that is, FR1, FR2, and FR3 of the human germline heavy chain IGHV1-3*01 and JH6 region (as FR4) of IGHJ6*01 were selected as the humanized antibody heavy chain framework regions; the light chain template was IGKV1-39*01/IGKJ4*01, that is, FR1, FR2, and FR3 of the human germline light chain IGKV1-39*01 and JK4 region (as FR4) of IGKJ4*01 were selected as the humanized antibody light chain framework regions. First, the CDR regions of the murine antibody mAb-99 were grafted onto the selected humanization template to replace the CDR regions of the humanization template, thus obtaining the humanized antibody variable region sequences of mAb-99. Then, the amino acid residues at positions 1, 24, 44, 71, and/or 91 (numbering according to Kabat numbering scheme) in the heavy chain variable region of the humanized antibody were subjected to back mutation, and the amino acid residues at positions 2, 4, 36, 38, 43, 44, and/or 58 (numbering according to Kabat numbering scheme) in the light chain variable region were subjected to back mutation, thus obtaining humanized antibody heavy/light chain variable regions. The specific sequences are as follows:
> Amino acid sequence of hAb99VH1 (Graft + Q1E, R71S) 
EVQLVQSGAEVKKPGASVKVSCKASGYTFTNYGINWVRQAPGQRLEWMGYIY
IGNGDIEYNAKFKGRVTITSDTSASTAYMELSSLRSEDTAVYYCARGRGLGHFDV
 
WGQGTTVTVSS
SEQ ID NO: 30
> Amino acid sequence of hAb99VH2 Graft + Q1E, R44G, R71S) 
EVQLVQSGAEVKKPGASVKVSCKASGYTFTNYGINWVRQAPGQGLEWMGYIY
IGNGDIEYNAKFKGRVTITSDTSASTAYMELSSLRSEDTAVYYCARGRLGHFDV
WGQGTTVTVSS
SEQ ID NO: 31
> Amino acid sequence of hAb99VH3 (Graft + Q1E, A24T, R44G, R71S, Y91F) 
EVQLVQSGAEVKKPGASVKVSCKTSGYTFTNYGINWVRQAPGQGLEWMGYIY
IGNGDIEYNAKFKGRVTITSDTSASTAYMELSSLRSEDTAVYFCARGRGLGHFDV
WGQGTTVTVSS
SEQ ID NO: 32
> Amino acid sequence of hAb99VL1 (Graft + P44N) 
DIQMTQSPSSLSASVGDRVTITCRASKSISKFLAWYQQKPGKANKLLIYSGSTLQ
SGVPSRFSGSGSGTDFTLTISSLQPEDFATYYCQQHNEYPWTFGGGTKVEIK
SEQ ID NO: 33
> Amino acid sequence of hAb99VL2 (Graft + Y36F, A43T, P44N) 
DIQMTQSPSSLSASVGDRVTITCRASKSISKFLAWFQQKPGKTNKLLIYSGSTLQS
GVPSRFSGSGSGTDFTLTISSLQPEDFATYYCQQHNEYPWTFGGGTKVEIK
SEQ ID NO: 34
> Amino acid sequence of hAb99VL3 (Graft + I2V, M4I, Y36F, A43T, P44N) 
DVQITQSPSSLSASVGDRVTITCRASKSISKFLAWFQQKPGKTNKLLIYSGSTLQS
GVPSRFSGSGSGTDFTLTISSLQPEDFATYYCQQHNEYPWTFGGGTKVEIK
SEQ ID NO: 35
> Amino acid sequence of hAb99VL4 (Graft + I2V, M4I, Y36F, Q38E, A43T, P44N, V58I)

DVQITQSPSSLSASVGDRVTITCRASKSISKFLAWFQEKPGKTNKLLIYSGSTLQS
GIPSRFSGSGSGTDFTLTISSLQPEDFATYYCQQHNEYPWTFGGGTKVEIK
SEQ ID NO: 36

In addition, amino residues at positions 6 and 7 of HCDR2 (YIYIGNGDIEYNAKFKG) in the antibody heavy chain variable region were mutated from NG to NV, NL, or QG to obtain new humanized antibody variable region sequences. The specific sequences are as follows:
> Amino acid sequence of hAb99VH1-NV 
EVQLVQSGAEVKKPGASVKVSCKASGYTFTNYGINWVRQAPGQRLEWMGYIY
IGNVDIEYNAKFKGRVTITSDTSASTAYMELSSLRSEDTAVYYCARGRLGHFDV
WGQGTTVTVSS
SEQ ID NO: 40
> Amino acid sequence of hAb99VH2-NV EVQLVQSGAEVKKPGASVKVSCKASGYTFTNYGINWVRQAPGQGLEWMGYIY 
IGNVDIEYNAKFKGRVTITSDTSASTAYMELSSLRSEDTAVYYCARGRLGHFDV
WGQGTTVTVSS
SEQ ID NO: 41
> Amino acid sequence of hAb99VH3-NV 
EVQLVQSGAEVKKPGASVKVSCKTSGYTFTNYGINWVRQAPGQGLEWMGYIY
IGNVDIEYNAKFKGRVTITSDTSASTAYMELSSLRSEDTAVYFCARGRGLGHFDV
WGQGTTVTVSS
SEQ ID NO: 42
> Amino acid sequence of hAb99VH1-NL 
EVQLVQSGAEVKKPGASVKVSCKASGYTFTNYGINWVRQAPGQRLEWMGYIY
IGNLDIEYNAKFKGRVTITSDTSASTAYMELSSLRSEDTAVYYCARGRGLGHFDV
WGQGTTVTVSS
SEQ ID NO: 43
> Amino acid sequence of hAb99VH2-NL 
EVQLVQSGAEVKKPGASVKVSCKASGYTFTNYGINWVRQAPGQGLEWMGYIY
IGNLDIEYNAKFKGRVTITSDTSASTAYMELSSLRSEDTAVYYCARGRGLGHFDV
WGQGTTVTVSS
SEQ ID NO: 44
> Amino acid sequence of hAb99VH3-NL 
EVQLVQSGAEVKKPGASVKVSCKTSGYTFTNYGINWVRQAPGQGLEWMGYIY
IGNLDIEYNAKFKGRVTITSDTSASTAYMELSSLRSEDTAVYFCARGRGLGHFDV
WGQGTTVTVSS
SEQ ID NO: 45
> Amino acid sequence of hAb99VH1-QG 
EVQLVQSGAEVKKPGASVKVSCKASGYTFTNYGINWVRQAPGQRLEWMGYIY
IGQGDIEYNAKFKGRVTITSDTSASTAYMELSSLRSEDTAVYYCARGRLGHFDV
WGQGTTVTVSS
SEQ ID NO: 46
> Amino acid sequence of hAb99VH2-QG 
EVQLVQSGAEVKKPGASVKVSCKASGYTFTNYGINWVRQAPGQGLEWMGYIY
IGQGDIEYNAKFKGRVTITSDTSASTAYMELSSLRSEDTAVYYCARGRGLGHFDV
WGQGTTVTVSS
SEQ ID NO: 47
> Amino acid sequence of hAb99VH3-QG

EVQLVQSGAEVKKPGASVKVSCKTSGYTFTNYGINWVRQAPGQGLEWMGYIY
IGQGDIEYNAKFKGRVTITSDTSASTAYMELSSLRSEDTAVYFCARGRGLGHFDV
WGQGTTVTVSS
SEQ ID NO: 48

The HCDR2 mutated sequences of the hAb99 heavy chain variable regions are shown in Table 6 below:

**Table 6. HCDR2 mutated sequences of hAb99 heavy chain variable regions**

| Heavy chain variable region | Variable region sequence number | Corresponding HCDR2 sequence (Kabat numbering scheme) |
|---|---|---|
| hAb99VH1-NV | SEQ ID NO: 40 | YIYIG**NV**DIEYNAKFKG (SEQ ID NO: 37) |
| hAb99VH2- NV | SEQ ID NO: 41 | YIYIG**NV**DIEYNAKFKG (SEQ ID NO: 37) |
| hAb99VH3- NV | SEQ ID NO: 42 | YIYIG**NV**DIEYNAKFKG (SEQ ID NO: 37) |
| hAb99VH1-NL | SEQ ID NO: 43 | YIYIG**NL**DIEYNAKFKG (SEQ ID NO: 38) |
| hAb99VH2- NL | SEQ ID NO: 44 | YIYIG**NL**DIEYNAKFKG (SEQ ID NO: 38) |
| hAb99VH3- NL | SEQ ID NO: 45 | YIYIG**NL**DIEYNAKFKG (SEQ ID NO: 38) |
| hAb99VH1-QG | SEQ ID NO: 46 | YIYIG**QG**DIEYNAKFKG (SEQ ID NO: 39) |
| hAb99VH2- QG | SEQ ID NO: 47 | YIYIG**QG**DIEYNAKFKG (SEQ ID NO: 39) |
| hAb99VH3- QG | SEQ ID NO: 48 | YIYIG**QG**DIEYNAKFKG (SEQ ID NO: 39) |

The above heavy chain variable regions were fused to the heavy chain constant region (set forth in SEQ ID NO: 22) and the light chain variable regions were fused to the light chain constant region (set forth in SEQ ID NO: 23) to construct anti-KLB antibodies, and the binding activity of the obtained antibodies for CHOK1-hKLB was detected (see Test Example 2 of the present disclosure for the experimental method). The experimental results are shown in Table 7.

**Table 7. Experimental results of binding of mAb-99 humanized antibodies to CHOK1-hKLB**

| **Antibody** | **Heavy chain variable region (VH)** | **Light chain variable region (VL)** | **EC50 (nM)** |
|---|---|---|---|
| hAb99-1 | SEQ ID NO: 30 | SEQ ID NO: 33 | 0.61 |
| hAb99-2 | SEQ ID NO: 30 | SEQ ID NO: 34 | 0.35 |
| hAb99-3 | SEQ ID NO: 30 | SEQ ID NO: 35 | 0.35 |
| hAb99-4 | SEQ ID NO: 30 | SEQ ID NO: 36 | 0.37 |
| hAb99-5 | SEQ ID NO: 31 | SEQ ID NO: 33 | 0.50 |
| hAb99-6 | SEQ ID NO: 31 | SEQ ID NO: 34 | 0.41 |
| hAb99-7 | SEQ ID NO: 31 | SEQ ID NO: 35 | 0.35 |
| hAb99-8 | SEQ ID NO: 31 | SEQ ID NO: 36 | 0.39 |
| hAb99-9 | SEQ ID NO: 32 | SEQ ID NO: 33 | 0.47 |
| hAb99-10 | SEQ ID NO: 32 | SEQ ID NO: 34 | 0.36 |
| hAb99-11 | SEQ ID NO: 32 | SEQ ID NO: 35 | 0.33 |
| hAb99-12 | SEQ ID NO: 32 | SEQ ID NO: 36 | 0.33 |
| hAb99-13 | SEQ ID NO: 40 | SEQ ID NO: 34 | 0.32 |
| hAb99-14 | SEQ ID NO: 43 | SEQ ID NO: 34 | 0.60 |
| hAb99-15 | SEQ ID NO: 46 | SEQ ID NO: 34 | 0.41 |
| hAb99-16 | SEQ ID NO: 41 | SEQ ID NO: 34 | 0.47 |
| hAb99-17 | SEQ ID NO: 44 | SEQ ID NO: 34 | 0.43 |
| hAb99-18 | SEQ ID NO: 47 | SEQ ID NO: 34 | 0.43 |
| hAb99-19 | SEQ ID NO: 42 | SEQ ID NO: 34 | 0.38 |
| hAb99-20 | SEQ ID NO: 45 | SEQ ID NO: 34 | 0.41 |
| hAb99-21 | SEQ ID NO: 48 | SEQ ID NO: 34 | 0.45 |
| mAb99-hIgG1 | SEQ ID NO: 2 | SEQ ID NO: 3 | 0.97 |

| | | | |
|---|---|---|---|
| Note: mAb99-hIgG1 is a chimeric antibody of mAb99, and its heavy chain constant region is set forth in SEQ ID NO: 22, and its light chain constant region is set forth in SEQ ID NO: 23. | | | |

Illustratively, the light and heavy chain sequences of the antibody hAb99-13 are as follows:
> Amino acid sequence of heavy chain of antibody hAb99-13 
EVQLVQSGAEVKKPGASVKVSCKASGYTFTNYGINWVRQAPGQRLEWMGYIY
IGNVDIEYNAKFKGRVTITSDTSASTAYMELSSLRSEDTAVYYCARGRGLGHFDV
WGQGTTVTVSSASTKGPSVFPLAPSSKSTSGGTAALGCLVKDYFPEPVTVSWNS
GALTSGVHTFPAVLQSSGLYSLSSVVTVPSSSLGTQTYICNVNHKPSNTKVDKKV
EPKSCDKTHTCPPCPAPEAAGGPSVFLFPPKPKDTLMISRTPEVTCVVVDVSHED
PEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLTCLVKGFYPSDI
AVEWESNGQPENNYKTTPPVLDSDGSFFLYSKLTVDKSRWQQGNVFSCSVMHE
ALHNHYTQKSLSLSPGK
SEQ ID NO: 78
> Amino acid sequence of light chain of antibody hAb99-13

DIQMTQSPSSLSASVGDRVTITCRASKSISKFLAWFQQKPGKTNKLLIYSGSTLQS
GVPSRFSGSGSGTDFTLTISSLQPEDFATYYCQQHNEYPWTFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQ ID NO: 79

### 2. Humanization of mAb-18

The template of the antibody mAb-18 humanized heavy chain was IGHV1-3*01/IGHJ6*01, that is, FR1, FR2, and FR3 of the human germline heavy chain IGHV1-3*01 and JH6 region (as FR4) of IGHJ6*01 were selected as the humanized antibody heavy chain framework regions; the light chain template was IGKV6-21*02/JGKJ2*01 or IGKV3-20*02/JGKJ2*01, that is, FR1, FR2, and FR3 of the human germline light chain IGKV6-21*02 or IGKV3-20*02 and JK2 region (as FR4) of JGKJ2*01 were selected as the humanized antibody light chain framework regions. First, the CDR regions of the murine antibody mAb-18 were grafted onto the selected humanization template to replace the CDR regions of the humanization template, thus obtaining the humanized antibody variable region sequences of mAb-18. In addition, the amino acid residues at positions 24, 48, 67, 69, 71, and/or 73 (numbering according to Kabat numbering scheme) in the heavy chain variable region of the humanized antibody were subjected to back mutation, the amino acid residue at position 1 (numbering according to Kabat numbering scheme) could also be subjected to mutation, and the amino acid residues at positions 2, 45, 47, 49, 58, and/or 71 (numbering according to Kabat numbering scheme) in the light chain variable region were subjected to back mutation, thus obtaining humanized antibody variable regions. The specific sequences are as follows:
> Amino acid sequence of hAb18VH1 (Graft + Q1E, R71V, T73K) 
EVQLVQSGAEVKKPGASVKVSCKASGYTFTDYAMHWVRQAPGQRLEWMGVI
STYSGTTNYNQNFKGRVTITVDKSASTAYMELSSLRSEDTAVYYCARSGPLDYW
GQGTTVTVSS
SEQ ID NO: 49
> Amino acid sequence of hAb18VH2 (Graft + Q1E, A24G, I69V, R71V, T73K) 
EVQLVQSGAEVKKPGASVKVSCKGSGYTFTDYAMHWVRQAPGQRLEWMGVI
STYSGTTNYNQNFKGRVTVTVDKSASTAYMELSSLRSEDTAVYYCARSGPLDY
WGQGTTVTVSS
SEQ ID NO: 50
> Amino acid sequence of hAb18VH3 (Graft + Q1E, A24G, M48I, V67A, I69V, R71V, T73K) 
EVQLVQSGAEVKKPGASVKVSCKGSGYTFTDYAMHWVRQAPGQRLEWIGVIS
TYSGTTNYNQNFKGRATVTVDKSASTAYMELSSLRSEDTAVYYCARSGPLDYW
GQGTTVTVSS
SEQ ID NO: 51
> Amino acid sequence of hAb18VL1 (Graft (IGKV6-21*02/JGKJ2*01) + L47W, K49Y) 
EIVLTQSPDFQSVTPKEKVTITCRASSSVSSSYLHWYQQKPDQSPKLWIYSTSNL
ASGVPSRFSGSGSGTDFTLTINSLEAEDAATYYCQQYSGYPLTFGQGTKLEIK
SEQ ID NO: 52
> Amino acid sequence of hAb18VL2 (Graft (IGKV6-21*02/JGKJ2*01) + I2N, L47W, K49Y, F71Y) 
ENVLTQSPDFQSVTPKEKVTITCRASSSVSSSYLHWYQQKPDQSPKLWIYSTSNL
ASGVPSRFSGSGSGTDYTLTINSLEAEDAATYYCQQYSGYPLTFGQGTKLEIK
SEQ ID NO: 53
> Amino acid sequence of hAb18VL3 (Graft (IGKV3-20*02/JGKJ2*01) + R45K, L47W, I58V) 
EIVLTQSPATLSLSPGERATLSCRASSSVSSSYLHWYQQKPGQAPKLWIYSTSNLA
SGVPARFSGSGSGTDFTLTISRLEPEDFAVYQQYSGYPLTFGQGTKLEIK
SEQ ID NO: 54
> Amino acid sequence of hAb18VL4 (Graft (IGKV3-20*02/JGKJ2*01) + I2N, R45K, L47W, I58V, F71Y)

ENVLTQSPATLSLSPGERATLSCRASSSVSSSYLHWYQQKPGQAPKLWIYSTSNL
ASGVPARFSGSGSGTDYTLTISRLEPEDFAVYQQYSGYPLTFGQGTKLEIK

SEQ ID NO: 55

In addition, the amino acid residues at positions 2, 3, 5, 6, 7, and 8 of LCDR1 (RASSSVSSSYLH (SEQ ID NO: 15)) and the amino acid residue at position 4 of LCDR2 (STSNLAS (SEQ ID NO: 16)) of the light chain variable region of hAb18VL2 were subjected to mutation to obtain new humanized antibody light chain variable region sequences. The specific sequences are as follows:
> Amino acid sequence of hAb 18VL2-1 
ENVLTQSPDFQSVTPKEKVTITCRASSSVSSSYLHWYQQKPDQSPKLWIYSTSQL
ASGVPSRFSGSGSGTDYTLTINSLEAEDAATYYCQQYSGYPLTFGQGTKLEIK
SEQ ID NO: 56
> Amino acid sequence of hAb 18VL2-2 
ENVLTQSPDFQSVTPKEKVTITCRATSSVSSSYLHWYQQKPDQSPKLWIYSTSQL
ASGVPSRFSGSGSGTDYTLTINSLEAEDAATYYCQQYSGYPLTFGQGTKLEIK
SEQ ID NO: 57
> Amino acid sequence of hAb 18VL2-3 
ENVLTQSPDFQSVTPKEKVTITCRVSSSVSSSYLHWYQQKPDQSPKLWIYSTSQL
ASGVPSRFSGSGSGTDYTLTINSLEAEDAATYYCQQYSGYPLTFGQGTKLEIK
SEQ ID NO: 58
> Amino acid sequence of hAb 18VL2-4 
ENVLTQSPDFQSVTPKEKVTITCRASSSVSTSYLHWYQQKPDQSPKLWIYSTSQL
ASGVPSRFSGSGSGTDYTLTINSLEAEDAATYYCQQYSGYPLTFGQGTKLEIK
SEQ ID NO: 59
> Amino acid sequence of hAb 18VL2-5 
ENVLTQSPDFQSVTPKEKVTITCRASSSVTSSYLHWYQQKPDQSPKLWIYSTSQL
ASGVPSRFSGSGSGTDYTLTINSLEAEDAATYYCQQYSGYPLTFGQGTKLEIK
SEQ ID NO: 60
> Amino acid sequence of hAb 18VL2-6 
ENVLTQSPDFQSVTPKEKVTITCRASSSLSSSYLHWYQQKPDQSPKLWIYSTSQL
ASGVPSRFSGSGSGTDYTLTINSLEAEDAATYYCQQYSGYPLTFGQGTKLEIK
SEQ ID NO: 61
> Amino acid sequence of hAb 18VL2-7 
ENVLTQSPDFQSVTPKEKVTITCRASSNVSSSYLHWYQQKPDQSPKLWIYSTSQL
ASGVPSRFSGSGSGTDYTLTINSLEAEDAATYYCQQYSGYPLTFGQGTKLEIK
SEQ ID NO: 62
> Amino acid sequence of hAb 18VL2-8 
ENVLTQSPDFQSVTPKEKVTITCRATSSVSSSYLHWYQQKPDQSPKLWIYSTSNL
ASGVPSRFSGSGSGTDYTLTINSLEAEDAATYYCQQYSGYPLTFGQGTKLEIK
SEQ ID NO: 63
> Amino acid sequence of hAb 18VL2-9 
ENVLTQSPDFQSVTPKEKVTITCRVSSSVSSSYLHWYQQKPDQSPKLWIYSTSNL
ASGVPSRFSGSGSGTDYTLTINSLEAEDAATYYCQQYSGYPLTFGQGTKLEIK
 SEQ ID NO: 64
> Amino acid sequence of hAb18VL2-10 
ENVLTQSPDFQSVTPKEKVTITCRASSSVSTSYLHWYQQKPDQSPKLWIYSTSNL
ASGVPSRFSGSGSGTDYTLTINSLEAEDAATYYCQQYSGYPLTFGQGTKLEIK
SEQ ID NO: 65
> Amino acid sequence of hAb 18VL2-11 
ENVLTQSPDFQSVTPKEKVTITCRASSSVTSSYLHWYQQKPDQSPKLWIYSTSNL
ASGVPSRFSGSGSGTDYTLTINSLEAEDAATYYCQQYSGYPLTFGQGTKLEIK
SEQ ID NO: 66
> Amino acid sequence of hAb18VL2-12 
ENVLTQSPDFQSVTPKEKVTITCRASSSLSSSYLHWYQQKPDQSPKLWIYSTSNL
ASGVPSRFSGSGSGTDYTLTINSLEAEDAATYYCQQYSGYPLTFGQGTKLEIK
SEQ ID NO: 67
> Amino acid sequence of hAb18VL2-13

ENVLTQSPDFQSVTPKEKVTITCRASSNVSSSYLHWYQQKPDQSPKLWIYSTSNL
ASGVPSRFSGSGSGTDYTLTINSLEAEDAATYYCQQYSGYPLTFGQGTKLEIK
SEQ ID NO: 68

The CDRs of the mutated sequences of the hAb18VL2 light chain variable regions are shown in Table 8 below:

**Table 8. CDR sequences of mutated sequences of hAb 18VL2 light chain variable regions**

| Light chain variable region | LCDR1 | LCDR2 | LCDR3 |
|---|---|---|---|
| hAb18VL2 | RASSSVSSSYLH (SEQ ID NO: 15) | STSNLAS (SEQ ID NO: 16) | QQYSGYPLT (SEQ ID NO: 17) |
| hAb18VL2-1 | RASSSVSSSYLH (SEQ ID NO: 15) | STSQLAS (SEQ ID NO: 75) | QQYSGYPLT (SEQ ID NO: 17) |
| hAb18VL2-2 | RATSSVSSSYLH (SEQ ID NO: 69) | STSQLAS (SEQ ID NO: 75) | QQYSGYPLT (SEQ ID NO: 17) |
| hAb18VL2-3 | RVSSSVSSSYLH (SEQ ID NO: 70) | STSQLAS (SEQ ID NO: 75) | QQYSGYPLT (SEQ ID NO: 17) |
| hAb18VL2-4 | RASSSVSTSYLH (SEQ ID NO: 71) | STSQLAS (SEQ ID NO: 75) | QQYSGYPLT (SEQ ID NO: 17) |
| hAb18VL2-5 | RASSSVTSSYLH (SEQ ID NO: 72) | STSQLAS (SEQ ID NO: 75) | QQYSGYPLT (SEQ ID NO: 17) |
| hAb18VL2-6 | RASSSLSSSYLH (SEQ ID NO: 73) | STSQLAS (SEQ ID NO: 75) | QQYSGYPLT (SEQ ID NO: 17) |
| hAb18VL2-7 | RASSNVSSSYLH (SEQ ID NO: 74) | STSQLAS (SEQ ID NO: 75) | QQYSGYPLT (SEQ ID NO: 17) |
| hAb18VL2-8 | RATSSVSSSYLH (SEQ ID NO: 69) | STSNLAS (SEQ ID NO: 16) | QQYSGYPLT (SEQ ID NO: 17) |
| hAb18VL2-9 | RVSSSVSSSYLH (SEQ ID NO: 70) | STSNLAS (SEQ ID NO: 16) | QQYSGYPLT (SEQ ID NO: 17) |
| hAb18VL2-10 | RASSSVSTSYLH (SEQ ID NO: 71) | STSNLAS (SEQ ID NO: 16) | QQYSGYPLT (SEQ ID NO: 17) |
| hAb18VL2-11 | RASSSVTSSYLH (SEQ ID NO: 72) | STSNLAS (SEQ ID NO: 16) | QQYSGYPLT (SEQ ID NO: 17) |
| hAb18VL2-12 | RASSSLSSSYLH (SEQ ID NO: 73) | STSNLAS (SEQ ID NO: 16) | QQYSGYPLT (SEQ ID NO: 17) |
| hAb18VL2-13 | RASSNVSSSYLH (SEQ ID NO: 74) | STSNLAS (SEQ ID NO: 16) | QQYSGYPLT (SEQ ID NO: 17) |

The above heavy chain variable regions were fused to the heavy chain constant region (set forth in SEQ ID NO: 22) and the light chain variable regions were fused to the light chain constant region (set forth in SEQ ID NO: 23) to construct anti-KLB antibodies, and the binding activity of the obtained antibodies for CHOK1-hKLB was detected (see Test Example 2 of the present disclosure for the experimental method). The experimental results are shown in Table 9.

**Table 9. Experimental results of binding activity of mAb-18 humanized antibodies for CHOK1-hKLB**

| **Antibody** | **Heavy chain variable region (VH)** | **Light chain variable region (VL)** | **EC50 (nM)** |
|---|---|---|---|
| hAb18-1 | SEQ ID NO: 49 | SEQ ID NO: 52 | 1.34 |
| hAb18-2 | SEQ ID NO: 49 | SEQ ID NO: 53 | 1.58 |
| hAb18-3 | SEQ ID NO: 50 | SEQ ID NO: 52 | 1.9 |
| hAb18-4 | SEQ ID NO: 50 | SEQ ID NO: 53 | 2 |
| hAb18-5 | SEQ ID NO: 51 | SEQ ID NO: 52 | 1.8 |
| hAb18-6 | SEQ ID NO: 51 | SEQ ID NO: 53 | 2 |
| hAb18-7 | SEQ ID NO: 49 | SEQ ID NO: 56 | 2.4 |
| hAb18-8 | SEQ ID NO: 49 | SEQ ID NO: 57 | 2.5 |
| hAb18-9 | SEQ ID NO: 49 | SEQ ID NO: 58 | 2.4 |
| hAb18-10 | SEQ ID NO: 49 | SEQ ID NO: 59 | 2.7 |
| hAb18-11 | SEQ ID NO: 49 | SEQ ID NO: 60 | 2.7 |
| hAb18-12 | SEQ ID NO: 49 | SEQ ID NO: 61 | 1.9 |
| hAb18-13 | SEQ ID NO: 49 | SEQ ID NO: 62 | 5.4 |
| hAb18-14 | SEQ ID NO: 49 | SEQ ID NO: 63 | 1.6 |
| hAb18-15 | SEQ ID NO: 49 | SEQ ID NO: 64 | 1.9 |
| hAb18-16 | SEQ ID NO: 49 | SEQ ID NO: 65 | 2.4 |
| hAb18-17 | SEQ ID NO: 49 | SEQ ID NO: 66 | 1.8 |
| hAb18-18 | SEQ ID NO: 49 | SEQ ID NO: 67 | 1.7 |
| hAb18-19 | SEQ ID NO: 49 | SEQ ID NO: 68 | 4.1 |
| mAb18-hIgG1 | SEQ ID NO: 4 | SEQ ID NO: 5 | 2.16 |

| | | | |
|---|---|---|---|
| Note: mAb18-hIgG1 is a chimeric antibody of mAb18, and its heavy chain constant region is set forth in SEQ ID NO: 22, and its light chain constant region is set forth in SEQ ID NO: 23. | | | |

Illustratively, the light and heavy chain sequences of the antibody hAb18-8 are as follows:
> Amino acid sequence of heavy chain of antibody hAb 18-8 
EVQLVQSGAEVKKPGASVKVSCKASGYTFTDYAMHWVRQAPGQRLEWMGVI
STYSGTTNYNQNFKGRVTITVDKSASTAYMELSSLRSEDTAVYYCARSGPLDYW
GQGTTVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVE
PKSCDKTHTCPPCPAPEAAGGPSVFLFPPKPKDTLMISRTPEVTCVVVDVSHEDP
EVKFNWYVDGVEVHNAKTKPREEQYNSTYRVVSVLTVLHQDWLNGKEYKCK
VSNKALPAPIEKTISKAKGQPREPQVYTLPPSRDELTKNQVSLTCLVKGFYPSDIA
VEWESNGQPENNYKTTPPVLDSDGSFFLYSKLTVDKSRWQQGNVFSCSVMHEA
LHNHYTQKSLSLSPGK
SEQ ID NO: 80
> Amino acid sequence of light chain of antibody hAb 18-8

ENVLTQSPDFQSVTPKEKVTITCRATSSVSSSYLHWYQQKPDQSPKLWIYSTSQL
ASGVPSRFSGSGSGTDYTLTINSLEAEDAATYYCQQYSGYPLTFGQGTKLEIKRT
VAAPSVFIFPPSDEQLKSGTASVVCLLNNFYPREAKVQWKVDNALQSGNSQESV
TEQDSKDSTYSLSSTLTLSKADYEKHKVYACEVTHQGLSSPVTKSFNRGEC
SEQ ID NO: 81

### Example 4. Preparation of Antibodies

### 1. Molecular cloning of antibodies

The designed antibody sequence was subjected to codon optimization to generate a coding gene sequence with human codon preference, a primer was designed, and VH/VK gene fragments of each antibody were constructed by PCR and homologously recombined with an expression vector pHr (with a signal peptide and a constant region gene (CH1-FC/CL) fragment). Thus, the antibody full-length expression plasmid VH-CH1-FC-pHr/VK-CL-pHr was constructed.

### 2. Expression and purification of antibodies

HEK293E cells were transfected with plasmids expressing heavy and light chains of the antibody at a ratio of 1:1.5, and after 6 days, expression supernatants were collected, centrifuged at high speed to remove impurities, and purified using a Protein A column. The column was washed with PBS until A₂₈₀ reading dropped to baseline. The target protein was eluted with 100 mM acetic acid (pH 3.0) and neutralized with 1 M Tris-HCl (pH 8.0). The eluted sample was appropriately concentrated and further purified by gel chromatography Superdex200 (GE) equilibrated with PBS to remove aggregates, and the eluate with monomer peak was collected and aliquoted for later use.

### Verification of technical effects of the antibodies of the present disclosure with biochemical test methods

### Test Example 1: Detection of Affinity of Anti-hKLB Antibodies by BIAcore

A Protein A biosensor chip (Cat. # 29127556, GE) was allowed to affinity capture an antibody, and then a certain concentration of hKLB (R&D, Cat. # 5889-KB) was allowed to flow through the surface of the chip. The reaction signals were detected in real time using a Biacore T200 instrument to obtain an association and dissociation curve. After dissociation was complete in each experiment cycle, the biosensor chip was washed with Glycine1.5 (Cat. # BR-1003-54, GE) for regeneration. The data were fitted using a 1:1 model to obtain antibody affinity data, and the specific experimental results are shown in Table 10 below.

**Table 10. Test results of affinity of test antibodies for hKLB**

| Antibody | ka (1/Ms) | kd (1/s) | KD (M) |
|---|---|---|---|
| hAb99-13 | 2.98E+05 | 6.26E-04 | 2.10E-09 |
| hAb18-8 | 1.06E+05 | 1.03E-04 | 9.77E-10 |
| hAb-23 | 2.47E+05 | 4.91E-05 | 1.99E-10 |

The experimental results show that the antibodies of the present disclosure have good binding function for human KLB.

### Test Example 2: Detection of Binding of Antibodies to KLB on Cell Membrane Surface by Flow Cytometry

The binding ability of the antibodies for CHOK1 cell strains CHOK1-hKLB and CHOK1-cynoKLB overexpressing human KLB and monkey KLB (see Example 1 for specific preparation procedure), respectively, was detected by flow cytometry. The specific procedure was as follows: the above cells were cultured in 10% FBS-containing IMDM medium and incubated in an incubator at 37 °C and 5% CO₂ for 2 days. The cells were then added to a cell plate at 1 × 10⁵ cells per well, centrifuged at 300 g for 5 min, and then washed once with 1% BSA. The antibody diluted in gradient was added to the cell plate at 100 µL per well, and the mixture was incubated at 4 °C for 1 h and washed once with 1% BSA. The diluted (1:400) APC-goat anti-human IgG Fc fluorescent secondary antibody was added at 100 µL per well, and the resulting mixture was incubated at 4 °C for 1 h. The plate was washed three times with 1% BSA, PBS was added at 100 µL per well, and the plate was read. The experimental results are shown in Table 11 below.

**Table 11. Experimental results of binding of antibodies to KLB on cell membrane surface**

| Antibody | EC₅₀ for binding to human KLB antigen (nM) | EC₅₀ for binding to monkey KLB antigen (nM) |
|---|---|---|
| hAb99-13 | 0.44±0.01 | 0.27±0.06 |
| hAb18-8 | 1.93±0.6 | 1.90±0.35 |
| hAb-23 | 0.39±0.09 | 0.13±0.04 |

The experimental results show that the antibodies hAb99-13, hAb18-8, and hAb-23 of the present disclosure can all bind to human KLB and monkey KLB on the surface of cell membrane with strong binding ability.

### Test Example 3: Competition of Antibodies and FGF19/FGF21 for Binding to KLB

Bio-FGF21/Bio-FGF19 (obtained by subjecting FGF21 and FGF19 to Biotin labeling with a Biotin Labeling Kit-NH2 (available from DOJINDO Molecular technologies Inc., LK03), wherein FGF21 is set forth in SEQ ID NO: 76 and FGF19 is set forth in SEQ ID NO: 77) was immobilized to an SA sensor (incubation for 80 s) at a concentration of 2 µg/mL, and the sensor was then transferred to a sample well containing KLB (R&D, 5889-KB-1 mg) or containing KLB + antibody premix (pre-incubation for at least 15 min) for 180 s of binding. The reaction signals were detected in real time using the ForteBio OCTET HTX instrument to obtain a binding curve. The experimental results are shown in Table 12 below.

**Table 12. Experiment on competitive binding of antibodies and FGF21 or FGF19**

| Group | FGF21 | FGF19 |
|---|---|---|
| hAb99-13+KLB | 108% | 99% |
| hAb18-8+KLB | 108% | 101% |
| hAb-23+KLB | 113% | 99% |
| KLB | 100% | 100% |

| | | |
|---|---|---|
| Note: the binding signal of KLB protein alone for Bio-FGF21 or Bio-FGF19 is defined as 100% | | |

The experimental results show that none of hAb99-13, hAb18-8, and hAb-23 of the present disclosure competes with FGF21 or FGF19 for binding to KLB.

### Test Example 4: Detection of Agonistic Activity of Antibodies for Recombinant Cell Line CHOK1-hKLB&hFGFR1c

The activation of the recombinant cell line CHOK1-hKLB&hFGFR1c (see Example 1) by the test antibodies was determined by *in vitro* assay. The luciferase reporter gene regulated by the GAL4 binding element and the GAL4-Elk1 fusion protein gene were transiently transfected into the recombinant cell line, and the activation activity was expressed as EC₅₀ value. The experimental procedures were as follows:
On day 1 of the experiment, CHOK1-hKLB&hFGFR1c cells were seeded into a 96-well plate (Corning, # 3903) at a density of 15000 cells/well using the DME/F12 medium containing 10% FBS, 10 µg/mL puromycin, and 800 µg/mL G418 to form 100 µL of cell suspension per well, and only 100 µL of PBS was added to the periphery of the 96-well plate. The plate was incubated overnight in a cell incubator at 37 °C and 5% CO₂. On day 2, the medium was discarded, and the starvation medium (DME/F12 without FBS) was added at 50 µL per well. The cells were transfected with pFA2-Elk1 and pFR-Luc at a ratio of 1:6 using Lipofectamine 3000, with 10 µL of a mixture of the plasmids and Lipofectamine 3000 added to each well. After transfection, the plate was incubated in an incubator at 37 °C and 5% CO₂ for 24 h. On day 3, the test antibody diluted in gradient with the starvation medium was added at 60 µL per well. The final concentration of the antibody was 9 concentration points obtained by 4-fold gradient dilution from 200 nM. The starvation medium was added to blank control wells. The plate was incubated in a cell incubator at 37 °C and 5% CO₂ for 24 h. On day 4, the 96-well cell culture plate was taken out, the One-glo Luciferase Assay System (a fluorescence detection reagent for signal activation, Promega, E6120) was added at 55 µL per well, and the luminescence signal value was read using a multi-functional microplate reader (EnVision2015, PerkinElmer). Curve fitting was performed using GraphPad Prism based on the logarithmic concentration and signal value of the antibody and EC₅₀ values were calculated. Activation fold = maximal signal value of sample/signal value of blank control; with regard to the degree of activation of KLB&FGFR1c receptor by the antibody at its highest concentration, a greater activation fold indicated a greater degree of activation. MK3655 (see anti-KLB antibody h5H23 in WO2015112886A2) was used in the experiment as a control, and the experimental results are shown in Table 13 below.

**Table 13. Experimental results of activation activity of antibodies for CHOK1-hKLB&hFGFR1c cells**

| Antibody | EC50 (nM) | Activation fold |
|---|---|---|
| hAb99-13 | 0.19 | 14.28 |
| MK3655 | 0.64 | 5.62 |

The experimental results show that the antibodies of the present disclosure have significant activation activity for CHOK1-hKLB&hFGFR1c cells, and the activation degree is stronger than that of the control molecule.

### Test Example 5: Detection of Agonistic Activity of Antibodies for HEK293T Expressing Human KLB and Human FGFR1c

The activation of HEK293T-hKLB&hFGFR1c (see Example 1), HEK293T cells transiently expressing human KLB and human FGFR1c, by the test antibodies was determined by *in vitro* cell assay. Human KLB, human FGFR1c, and Luciferase reporter gene regulated by the phosphorylated Elk were transiently transfected into the cell line, and the activation activity was expressed as EC₅₀ value. The specific procedures were as follows:
On day 1 of the experiment, HEK293T cells were seeded into a 96-well plate (Corning BioCoat, # 356692) at a density of 40000 cells/well using the DMEM/HIGH GLUCOSE medium containing 10% FBS to form 100 µL of cell suspension per well, and only 100 µL of PBS was added to the periphery of the 96-well plate. The plate was incubated overnight in a cell incubator at 37 °C and 5% CO₂. On day 2, the original medium was discarded, and the starvation medium (DMEM/HIGH GLUCOSE without FBS) was added at 50 µL per well. The cells were transfected with SRE-Luc2P (Promega, E1340), hKLB (see Uniprot ID: Q86Z14 for the sequence), and hFGFR1c (see Uniprot ID: P11362 for the sequence) at a ratio of 30:9:1 using Lipofectamine 3000, with 10 µL of a mixture of the plasmids and Lipofectamine 3000 added to each well. After transfection, the plate was incubated in an incubator at 37 °C and 5% CO₂ for 24 h. On day 3, the test antibody diluted in gradient with the starvation medium was added at 60 µL per well. The final concentration of the antibody was 9 concentration points obtained by 4-fold gradient dilution from 200 nM. The starvation medium was added to blank control wells. The plate was incubated in a cell incubator at 37 °C and 5% CO₂ for 24 h. On day 4, the 96-well cell culture plate was taken out, the One-glo Luciferase Assay System (Promega, E6120) was added at 60 µL per well, and the luminescence signal value was read using a multi-functional microplate reader (EnVision2015, PerkinElmer). Curve fitting was performed using GraphPad Prism based on the logarithmic concentration and signal value of the antibody and EC₅₀ values were calculated. Activation fold = maximal signal value of sample/signal value of blank control; with regard to the degree of activation of KLB&FGFR1c receptor by the antibody at its highest concentration, a greater activation fold indicated a greater degree of activation. The experimental results are shown in Table 14.

**Table 14. Detection results of agonistic activity of antibodies for recombinant cell line HEK293 T-hKLB&hFGFR1c**

| Antibody | EC50 (nM) | Activation fold |
|---|---|---|
| hAb99-13 | 2.62 | 3.57 |
| hAb18-8 | 5.35 | 2.29 |
| MK3655 | 5.06 | 1.65 |

The experimental results show that the antibodies of the present disclosure have significant activation activity for HEK293T-hKLB&hFGFR1c and the activation activity is stronger than that of the control molecule.

### Test Example 6: Detection of Activation of Recombinant Cell Line HEK293-hFGFR1c by Antibodies

To verify that the activation of human KLB and human FGFR1c stable cell lines by the antibodies is human KLB-dependent, this test example determined the activation of HEK293-hFGFR1c, HEK293 (ATCC, CRL-1573) cells stably expressing human FGFR1c (see Uniprot ID: P11362 for the sequence) by the test antibodies. The specific procedures were as follows:
On day 1 of the experiment, HEK293-hFGFR1c cells were seeded into a 96-well plate (Corning, # 3903) at a density of 25000 cells/well using the DMEM/HIGH GLUCOSE medium containing 10% FBS to form 100 µL of cell suspension per well, and only 100 µL of PBS was added to the periphery of the 96-well plate. The plate was incubated overnight in a cell incubator at 37 °C and 5% CO₂. On day 2, the cells were transfected with SRE-Luc2P using Lipofectamine 3000, with 10 µL of a mixture of the plasmid and Lipofectamine 3000 added to each well. After transfection, the plate was incubated in an incubator at 37 °C and 5% CO₂ for 24 h. On day 3, the test antibody diluted in gradient with the plating medium was added at 10 µL per well. The final concentration of the antibody was the ninth concentration point obtained by 4-fold gradient dilution from 200 nM. The plating medium was added to blank control wells. The plate was incubated in a cell incubator at 37 °C and 5% CO₂ for 16 h. On day 4, the 96-well cell culture plate was taken out, the One-glo Luciferase Assay System (a fluorescence detection reagent for signal activation, Promega, E6120) was added at 60 µL per well, and the luminescence signal value was read using a multi-functional microplate reader (EnVision2015, PerkinElmer). The ratio of the signal value of the test antibody to the reading of the blank control represented the activation activity of the antibody on FGFR1c, and activation fold = maximal signal value of sample/signal value of blank control; with regard to the degree of activation of HEK293-hFGFR1c by the antibody at the highest sample concentration, a greater activation fold indicated a greater degree of activation. FGF2 (Sino Biological, 10014-HNAE) was used as a control in the experiment. The experimental results are shown in Table 15.

**Table 15. Detection results of activation activity of antibodies for HEK293-hFGFR1c cells**

| Monoclonal antibody | Activation fold |
|---|---|
| hAb99-13 | 0.77 |
| hAb18-8 | 0.81 |
| hAb-23 | 0.86 |
| FGF2 | 11.59 |

The experimental results show that the antibodies of the present disclosure have weak activation activity on HEK293-hFGFR1c, indicating that the activation of human KLB and human FGFR1c stable cell lines by the antibodies of the present disclosure is human KLB-dependent.

### Test Example 7: Determination of Effect of Antibodies on ERK Phosphorylation Level of L6/hKLB Cells Expressing FGFR2c/FGFR3c/FGFR4

By determining the effect of the test antibodies in increasing ERK phosphorylation level of the L6/hKLB cell strain expressing human FGFR2c, FGFR3c, or FGFR4 in an *in vitro* cell assay, the selectivity of the antibodies for FGFR2c, FGFR3c, or FGFR4 was determined. The specific procedures were as follows:
Firstly, an L6-hKLB monoclonal cell strain (L6 cells were derived from ATCC, CRL-1458) stably expressing human KLB (see Uniprot ID: Q86Z14 for the sequence) was constructed by lentivirus infection, and then L6-hKLB&hFGFR2c, L6-hKLB&hFGFR3c, and L6-hKLB&hFGFR4 cells stably expressing human FGFR2c (human FGF receptor 2c), human FGFR3c (human FGF receptor 3c), and human FGFR4 (human FGF receptor 4), respectively, were constructed by lentivirus infection. On day 1 of the experiment, the L6-hKLB&hFGFR2c, L6-hKLB&hFGFR3c, and L6-hKLB&hFGFR4 cells were each seeded into a 96-well plate at a density of 25000 cells per well using DMEM/HIGH GLUCOSE medium (GE, SH30243.01) containing 10% FBS (Gibco, 10099-141C) to form 100 µL of cell suspension per well. The plate was incubated overnight in a cell incubator at 37 °C and 5% CO₂. On day 2, the complete medium in the plate was shaken off, and DMEM/HIGH GLUCOSE medium containing 0.1% Fatty-free BSA (Sangon Biotech, A602448) was added at 90 µL per well. The plate was incubated in an incubator at 37 °C and 5% CO₂ for 1 h. Then, the test antibody diluted in gradient with PBS was added at 10 µL per well. The final concentration of the antibody was 9 concentration points obtained by 4-fold gradient dilution from 50 nM or 500 nM. Blank control wells without the antibody were provided. The plate was incubated in an incubator at 37 °C and 5% CO₂ for 15 min. The levels of pERK and total ERK in cell lysates were determined using the Advanced ERK phospho-T202 /Y204 kit (Cisbio, 64AERPEH) and ERK total Kit (Cisbio, 64NRKPEH) according to the procedures specified in the instructions. The fluorescence values at an excitation wavelength of 337 nm and at emission wavelengths of 665 nm and 620 nm were read using a PHERAstar multifunctional microplate reader (BMG Labtech). The pERK and total ERK ratio corresponding to each concentration of the compound was calculated according to the following formula: ratio = signal value_{665 nm}/signal value_{620 nm}. The ratio of pERK/total ERK of the antibody at its highest concentration was divided by the ratio of the blank control well to calculate the S/N value, i.e., the activation fold, and a greater activation fold indicated a greater degree of activation. Activation activity of the antibodies of the present disclosure for the ERK phosphorylation level was determined by the above detection, and AKR001 (see FGF21 analog of SEQ ID NO: 47 in WO2010129503A1) was used as a control. The experimental results are shown in Table 16 below.

**Table 16. Detection results of activation of ERK phosphorylation level of cells by antibodies**

| Antibody | Activation fold | | |
|---|---|---|---|
| | L6-hKLB&hFGFR2c | L6-hKLB&hFGFR3c | L6-hKLB&hFGFR4 |
| hAb99-13 | 1.38±0.28 | 4.95±0.81 | 1.54±0.20 |
| hAb18-8 | 1.08±0.13 | 1.41±0.21 | 0.98±0.06 |
| hAb-23 | 0.93±0.05 | 1.08±0.19 | 0.91±0.04 |
| AKR001 | 5.81±0.41 | 10.19±2.20 | 2.11±0.49 |

The experimental results show that the activation of L6-hKLB&hFGFR2c, L6-hKLB&hFGFR3c, and L6-hKLB&hFGFR4 cells by the antibodies of the present disclosure is weaker than that by the control molecule AKR001.

### Test Example 8: In Vivo Efficacy Experiment of Antibodies

In this experiment, the spontaneous nonalcoholic steatohepatitis (NASH) model of rhesus monkey was selected, and the effect of antibodies on liver lipid content, NAS (NAFLD activity score), body weight, BMI (body mass index), abdominal girth, and glycolipid metabolism was evaluated using MRI imaging technique and liver biopsy technique.

Experimental procedures: NASH rhesus monkeys were subjected to adaptive feeding for 35 days, during which glycolipid metabolism indexes of the rhesus monkeys were detected, and liver biopsy was carried out under ultrasonic guidance for pathological analysis. The intrahepatic lipid content was quantitatively analyzed by using MRI (GE, 3T), and the rhesus monkeys with stable intrahepatic lipid content indexes were selected and averagely divided into three groups, namely hAb99-13 group (13 mg/kg), hAb18-8 group (13 mg/kg), and placebo group (vehicle control group, 10 mM glacial acetic acid, 9% sucrose, pH 5.2). For each group, the drug was administrated by intravenous injection once every 2 weeks, and a total of 5 injections were given. After administration, the intrahepatic lipid content was quantitatively analyzed by MRI (GE, 3T), liver biopsy was carried out under GE ultrasound guidance, and the sitting height and abdominal girth of the rhesus monkeys before and after administration were examined; the glycolipid metabolism, liver function indexes, and body weight of the rhesus monkeys were periodically detected and the average value was calculated. The experimental results are shown in Table 17-1 to Table 17-10.

The experimental results of intrahepatic lipid content are shown in Table 17-1, and the experimental results show that in the placebo group, the percentage of ROI (region of interest) changed significantly before and 60 days after the administration (D60); in the hAb99-13 group, the ROI% value decreased significantly from 8.4% before administration (D0) to 6.6% at day 60 after the administration (P < 0.05).

**Table 17-1. Detection results of effect of antibody on liver lipid content of NASH rhesus monkeys**

| Group | ROI before administration (%) | D60 ROI (%) | Change rate (%) |
|---|---|---|---|
| hAb99-13 group | 8.4 | 6.6 | -20.84 |
| Placebo group | 7.3 | 7.4 | 0.62 |

| | | | |
|---|---|---|---|
| Note: in the table, change rate = (current value - baseline value)/baseline value * 100%, wherein the baseline value is the ROI (%) before administration, and the current value is the ROI (%) at the time point of detection (D60) | | | |

The pathological analysis results for liver biopsy are shown in Table 17-2, and the experimental results show that in the placebo group, the pathological results of the liver tissues before and after the administration didn't show significant amelioration; in the hAb99-13 administration group, compared with the value before administration, the average NAS score decreased from 3.7 to 2.7 at day 65 after administration, and the steatosis score also decreased significantly.

**Table 17-2. Effect of antibody on hepatic pathology of NASH rhesus monkeys**

| Group | Steatosis score | | NAS score | |
|---|---|---|---|---|
| | D0 | D65 | D0 | D65 |
| hAb99-13 group | 2 | 0.6 | 3.7 | 2.7 |
| Placebo group | 2 | 2 | 3.3 | 3.3 |

| | | | | |
|---|---|---|---|---|
| Note: D0 refers to the value detected before administration, and D65 refers to the value detected on the day 65 after the first administration. | | | | |

The experimental results of the effect of the antibodies on islet function are shown in Table 17-3 and Table 17-4. The experimental results show that AUC_{Ins0-30min} decreased in each experimental group, and the decrease rate in the hAb99-13 group and the hAb18-8 group exceeded 50%; in the hAb99-13 group and the hAb18-8 group, HOMA-IR (homeostatic model assessment for insulin resistance index, used for assessing insulin resistance level, increased with increasing insulin resistance level) showed a relatively great decrease after administration.

**Table 17-3. Effect of antibodies on IVGTT insulin index of NASH rhesus monkeys**

| Group | AUC_{Ins0-30rnin} (U*min/L) | | |
|---|---|---|---|
| | Before administration | D65 | Change rate (%) |
| hAb99-13 group | 9.81 | 4.22 | -53.39 |
| hAb18-8 group | 4.74 | 2.10 | -54.24 |
| Placebo group | 6.26 | 4.92 | -22.97 |

| | | | |
|---|---|---|---|
| Note: change rate = (current value - baseline value)/baseline value * 100%, wherein the baseline value is the AUC_{Ins0-30min} (U*min/L) before administration, and the current value is the AUC_{Ins0-30min} (U*min/L) at the time point of detection (D65) | | | |

**Table 17-4. Experimental results of effect of antibodies on HOMA-IR change rate (%) of NASH rhesus monkeys**

| Group | Before administration | D14 | D28 | D35 | D49 |
|---|---|---|---|---|---|
| hAb99-13 group | 0.00 | -25.15 | -15.72 | -31.16 | -52.41 |
| hAb18-8 group | 0.00 | -40.75 | -35.15 | -48.31 | -49.26 |
| Placebo group | 0.00 | 25.11 | 11.34 | 103.23 | 57.79 |

| | | | | | |
|---|---|---|---|---|---|
| Note: HOMA-IR change rate = (current value - baseline value)/baseline value * 100%, wherein the baseline value is the HOMA-IR before administration, and the current value is the HOMA-IR at the time point of detection. | | | | | |

The experimental results of the effect of the antibodies on fasting plasma glucose (FPG) and fasting plasma insulin (FPI) of NASH rhesus monkeys are shown in Table 17-5 and Table 17-6. The experimental results show that in the placebo group, the FPG and FPI of the animals didn't change significantly before and after administration; the FPG and FPI of the antibody administration groups showed a significant decrease.

**Table 17-5. Experimental results of effect of antibodies on FPG of NASH rhesus monkeys**

| Group | FPG change rate (%) | | | | | |
|---|---|---|---|---|---|---|
| | D0 | D7 | D21 | D42 | D49 | D56 |
| hAb99-13 group | 0.00 | -8.14 | -14.34 | -18.47 | -20.55 | -16.80 |
| hAb18-8 group | 0.00 | -3.34 | -9.67 | -14.47 | -18.20 | -10.49 |
| Placebo group | 0.00 | -2.36 | -0.15 | -3.59 | -2.03 | 0.74 |

| | | | | | | |
|---|---|---|---|---|---|---|
| Note: D0 represents before administration, and D7 represents day 7 after the first administration; FPG change rate = (current value - baseline value)/baseline value * 100%, wherein the baseline value is the FPG value before administration, and the current value is the FPG value at the time point of detection. | | | | | | |

**Table 17-6. Experimental results of effect of antibody on FPI of NASH rhesus monkeys**

| Group | FPI change rate (%) | | | | |
|---|---|---|---|---|---|
| | D0 | D7 | D42 | D49 | D56 |
| hAb99-13 group | 0.00 | -3.98 | -27.63 | -37.33 | -52.79 |
| hAb18-8 | 0.00 | -3.37 | -51.53 | -39.14 | -62.92 |
| Placebo group | 0.00 | -11.19 | -6.36 | 54.10 | -13.50 |

| | | | | | |
|---|---|---|---|---|---|
| Note: D0 represents before administration, and D7 represents day 7 after the first administration; change rate = (current value - baseline value)/baseline value * 100%, wherein the baseline value is the FPI value before administration, and the current value is the FPI value at the time point of detection. | | | | | |

The experimental results of the effect of the antibody on TG of NASH rhesus monkeys are shown in Table 17-7. The experimental results show that in the placebo group, there was no significant change of TG; there was a certain decrease in TG in the hAb99-13 administration group.

**Table 17-7. Effect of antibodies on TG of NASH rhesus monkeys**

| Group | TG change rate (%) | | | | | | |
|---|---|---|---|---|---|---|---|
| | D0 | D7 | D14 | D28 | D35 | D49 | D56 |
| hAb99-13 group | 0.00 | -28.17 | -16.72 | -22.54 | -28.38 | -33.58 | -24.55 |
| Placebo group | 0.00 | -7.02 | 19.43 | 16.88 | 22.07 | 48.01 | 13.19 |

| | | | | | | | |
|---|---|---|---|---|---|---|---|
| Note: D0 represents before administration, and D7 represents day 7 after the first administration; change rate = (current value - baseline value)/baseline value * 100%, wherein the baseline value is the TG value before administration, and the current value is the TG value at the time point of detection. | | | | | | | |

The effect of the antibodies on adiponectin of NASH rhesus monkeys is shown in Table 17-8, and the experimental results show that adiponectin of animals in the placebo group showed a descending trend; adiponectin showed a certain degree of increase in the administration groups of the antibodies of the present disclosure.

**Table 17-8. Effect of antibodies on adiponectin of NASH rhesus monkeys**

| Group | Adiponectin change rate (%) | | | | | | |
|---|---|---|---|---|---|---|---|
| | D0 | D7 | D14 | D21 | D28 | D35 | D49 |
| hAb99-13 group | 0.00 | 0.21 | 1.47 | 5.73 | 8.63 | 6.99 | 14.48 |
| hAb18-8 group | 0.00 | 2.28 | 8.70 | 8.90 | 13.76 | 19.50 | 35.53 |
| Placebo group | 0.00 | -14.50 | -19.41 | -14.84 | -17.07 | -15.28 | -12.11 |

| | | | | | | | |
|---|---|---|---|---|---|---|---|
| Note: D0 represents before administration, and D7 represents day 7 after the first administration; change rate = (current value - baseline value)/baseline value * 100%, wherein the baseline value is the adiponectin value before administration, and the current value is the adiponectin value at the time point of detection. | | | | | | | |

The experimental results of the effect of the antibodies on abdominal girth and BMI of rhesus monkeys are shown in Table 17-9 and Table 17-10. The experimental results show that in the placebo group, the abdominal girth and BMI of the animals didn't change significantly; the abdominal girth and BMI in the antibody administration groups showed a significant decrease.

**Table 17-9. Effect of antibodies on abdominal girth of NASH rhesus monkeys**

| Group | Abdominal girth (cm) | | Change rate (%) |
|---|---|---|---|
| | Before administration | D60 | |
| hAb99-13 group | 62.3 | 50.8 | -18.6 |
| hAb18-8 group | 59.9 | 48.2 | -19.6 |
| Placebo group | 58.8 | 56.4 | -4.0 |

| | | | |
|---|---|---|---|
| Note: change rate = current value - baseline value)/baseline value * 100%, wherein the baseline value is the value detected before administration, and the current value is the value detected at the time point of detection after administration (D60) | | | |

**Table 17-10. Effect of antibodies on BMI of NASH rhesus monkeys**

| Group | BMI (kg/m²) | | Change rate (%) |
|---|---|---|---|
| | Before administration | D60 | |
| hAb99-13 group | 50 | 43 | -15 |
| hAb 18-8 group | 45 | 37 | -22 |
| Placebo group | 44 | 43 | -2 |

| | | | |
|---|---|---|---|
| Note: BMI = body weight/sitting height; change rate = (current value - baseline value)/baseline value * 100%, wherein the baseline value is the value detected before administration, and the current value is the value detected at the time point of detection after administration (D60). | | | |

Although the foregoing invention has been described in detail by way of drawings and examples for purposes of clarity of understanding, the description and examples should not be construed as limiting the scope of the present disclosure. The disclosures of all patents and scientific literature cited herein are clearly incorporated by reference in their entirety.

## Claims

1. An anti-KLB antibody, comprising a heavy chain variable region and a light chain variable region, wherein the heavy chain variable region comprises a HCDR1, a HCDR2, and a HCDR3, and the light chain variable region comprises a LCDR1, a LCDR2, and a LCDR3, wherein
i) the HCDR1, the HCDR2, and the HCDR3 of the heavy chain variable region comprise amino acid sequences of a HCDR1, a HCDR2, and a HCDR3, respectively, in any one of SEQ ID NOs: 40-48, and the LCDR1, the LCDR2, and the LCDR3 of the light chain variable region comprise amino acid sequences of a LCDR1, a LCDR2, and a LCDR3, respectively, in SEQ ID NO: 34; or, the HCDR1, the HCDR2, and the HCDR3 of the heavy chain variable region comprise amino acid sequences of a HCDR1, a HCDR2, and a HCDR3, respectively, in SEQ ID NO: 2, and the LCDR1, the LCDR2, and the LCDR3 of the light chain variable region comprise amino acid sequences of a LCDR1, a LCDR2, and a LCDR3, respectively, in SEQ ID NO: 3; or
ii) the HCDR1, the HCDR2, and the HCDR3 of the heavy chain variable region comprise amino acid sequences of a HCDR1, a HCDR2, and a HCDR3, respectively, in SEQ ID NO: 49, and the LCDR1, the LCDR2, and the LCDR3 of the light chain variable region comprise amino acid sequences of a LCDR1, a LCDR2, and a LCDR3, respectively, in any one of SEQ ID NOs: 56-68; or, the HCDR1, the HCDR2, and the HCDR3 of the heavy chain variable region comprise amino acid sequences of a HCDR1, a HCDR2, and a HCDR3, respectively, in SEQ ID NO: 4, and the LCDR1, the LCDR2, and the LCDR3 of the light chain variable region comprise amino acid sequences of a LCDR1, a LCDR2, and a LCDR3, respectively, in SEQ ID NO: 5; or
iii) the HCDR1, the HCDR2, and the HCDR3 of the heavy chain variable region comprise amino acid sequences of a HCDR1, a HCDR2, and a HCDR3, respectively, in SEQ ID NO: 18, and the LCDR1, the LCDR2, and the LCDR3 of the light chain variable region comprise amino acid sequences of a LCDR1, a LCDR2, and a LCDR3, respectively, in SEQ ID NO: 19.

2. The anti-KLB antibody according to claim 1, wherein:
i) in the heavy chain variable region, the HCDR1 comprises the amino acid sequence of SEQ ID NO: 6, the HCDR2 comprises the amino acid sequence of SEQ ID NO: 37, 38, 39, or 7, and the HCDR3 comprises the amino acid sequence of SEQ ID NO: 8; and in the light chain variable region, the LCDR1 comprises the amino acid sequence of SEQ ID NO: 9, the LCDR2 comprises the amino acid sequence of SEQ ID NO: 10, and the LCDR3 comprises the amino acid sequence of SEQ ID NO: 11; or
ii) in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14; and in the light chain variable region, the LCDR1 comprises the amino acid sequence of SEQ ID NO: 69, 15, 70, 71, 72, 73, or 74, the LCDR2 comprises the amino acid sequence of SEQ ID NO: 75 or 16, and the LCDR3 comprises the amino acid sequence of SEQ ID NO: 17; or
iii) in the heavy chain variable region, the HCDR1 comprises the amino acid sequence of SEQ ID NO: 24, the HCDR2 comprises the amino acid sequence of SEQ ID NO: 25, and the HCDR3 comprises the amino acid sequence of SEQ ID NO: 26; and in the light chain variable region, the LCDR1 comprises the amino acid sequence of SEQ ID NO: 27, the LCDR2 comprises the amino acid sequence of SEQ ID NO: 28, and the LCDR3 comprises the amino acid sequence of SEQ ID NO: 29;
preferably,
i) in the heavy chain variable region, the HCDR1 comprises the amino acid sequence of SEQ ID NO: 6, the HCDR2 comprises the amino acid sequence of SEQ ID NO: 37, and the HCDR3 comprises the amino acid sequence of SEQ ID NO: 8; and in the light chain variable region, the LCDR1 comprises the amino acid sequence of SEQ ID NO: 9, the LCDR2 comprises the amino acid sequence of SEQ ID NO: 10, and the LCDR3 comprises the amino acid sequence of SEQ ID NO: 11; or
ii) in the heavy chain variable region, the HCDR1 comprises the amino acid sequence of SEQ ID NO: 12, the HCDR2 comprises the amino acid sequence of SEQ ID NO: 13, and the HCDR3 comprises the amino acid sequence of SEQ ID NO: 14; and in the light chain variable region, the LCDR1 comprises the amino acid sequence of SEQ ID NO: 69, the LCDR2 comprises the amino acid sequence of SEQ ID NO: 75, and the LCDR3 comprises the amino acid sequence of SEQ ID NO: 17.

3. The anti-KLB antibody according to claim 1 or 2, being a murine antibody, a chimeric antibody, a humanized antibody, or a fully human-derived antibody.

4. The anti-KLB antibody according to any one of claims 1 to 3, wherein
i) the heavy chain variable region comprises SEQ ID NO: 40, 30, 31, 32, 41, 42, 43, 44, 45, 46, 47, or 48, or an amino acid sequence having at least 90% sequence identity thereto, and the light chain variable region comprises SEQ ID NO: 34, 33, 35, or 36, or an amino acid sequence having at least 90% sequence identity thereto; or
ii) the heavy chain variable region comprises SEQ ID NO: 49, 50, or 51, or an amino acid sequence having at least 90% sequence identity thereto, and the light chain variable region comprises SEQ ID NO: 57, 52, 53, 54, 55, 56, 58, 59, 60, 61, 62, 63, 64, 65, 66, 67, or 68, or an amino acid sequence having at least 90% sequence identity thereto; or
iii) the heavy chain variable region comprises SEQ ID NO: 18 or an amino acid sequence having at least 90% sequence identity thereto, and the light chain variable region comprises SEQ ID NO: 19 or an amino acid sequence having at least 90% sequence identity thereto; or
iv) the heavy chain variable region comprises SEQ ID NO: 2 or an amino acid sequence having at least 90% sequence identity thereto, and the light chain variable region comprises SEQ ID NO: 3 or an amino acid sequence having at least 90% sequence identity thereto; or
v) the heavy chain variable region comprises SEQ ID NO: 4 or an amino acid sequence having at least 90% sequence identity thereto, and the light chain variable region comprises SEQ ID NO: 5 or an amino acid sequence having at least 90% sequence identity thereto.

5. The anti-KLB antibody according to any one of claims 1 to 4, wherein
i) the heavy chain variable region comprises SEQ ID NO: 40 or an amino acid sequence having at least 90% sequence identity thereto, and the light chain variable region comprises SEQ ID NO: 34 or an amino acid sequence having at least 90% sequence identity thereto; preferably, the heavy chain variable region comprises, in a framework region thereof, one or more amino acid back mutations selected from the group consisting of 1E, 24T, 44G, 71S, and 91F (numbering according to Kabat numbering scheme), and the light chain variable region comprises, in a framework region thereof, one or more amino acid back mutations selected from the group consisting of 2V, 4I, 36F, 38E, 43T, 44N, and 58I (numbering according to Kabat numbering scheme);
ii) the heavy chain variable region comprises SEQ ID NO: 49 or an amino acid sequence having at least 90% sequence identity thereto, and the light chain variable region comprises SEQ ID NO: 57 or an amino acid sequence having at least 90% sequence identity thereto; preferably, the heavy chain variable region comprises, in a framework region thereof, one or more amino acid back mutations selected from the group consisting of 1E, 24G, 48I, 67A, 69V, 71V, and 73K (numbering according to Kabat numbering scheme), and/or the light chain variable region comprises, in a framework region thereof, one or more amino acid back mutations selected from the group consisting of 2N, 45K, 47W, 49Y, 58V, and 71Y (numbering according to Kabat numbering scheme); or
iii) the heavy chain variable region comprises SEQ ID NO: 18 or an amino acid sequence having at least 90% sequence identity thereto, and the light chain variable region comprises SEQ ID NO: 19 or an amino acid sequence having at least 90% sequence identity thereto.

6. The anti-KLB antibody according to any one of claims 1 to 5, comprising a heavy chain constant region and a light chain constant region, wherein
preferably, the heavy chain constant region is a human IgG1 heavy chain constant region, and the light chain constant region is a human κ light chain constant region;
more preferably, the heavy chain constant region comprises the amino acid sequence of SEQ ID NO: 22, and the light chain constant region comprises the amino acid sequence of SEQ ID NO: 23.

7. The anti-KLB antibody according to claim 6, wherein the anti-KLB antibody comprises a heavy chain and a light chain, wherein:
i) the heavy chain comprises an amino acid sequence having at least 90% sequence identity to SEQ ID NO: 78, and the light chain comprises an amino acid sequence having at least 90% sequence identity to SEQ ID NO: 79; or
ii) the heavy chain comprises an amino acid sequence having at least 90% sequence identity to SEQ ID NO: 80, and the light chain comprises an amino acid sequence having at least 90% sequence identity to SEQ ID NO: 81; or
iii) the heavy chain comprises an amino acid sequence having at least 90% sequence identity to SEQ ID NO: 20, and the light chain comprises an amino acid sequence having at least 90% sequence identity to SEQ ID NO: 21.

8. The anti-KLB antibody according to claim 6, wherein the anti-KLB antibody comprises a heavy chain and a light chain, wherein:
i) the heavy chain comprises the amino acid sequence of SEQ ID NO: 78, and the light chain comprises the amino acid sequence of SEQ ID NO: 79;
ii) the heavy chain comprises the amino acid sequence of SEQ ID NO: 80, and the light chain comprises the amino acid sequence of SEQ ID NO: 81; or
iii) the heavy chain comprises the amino acid sequence of SEQ ID NO: 20, and the light chain comprises the amino acid sequence of SEQ ID NO: 21.

9. An isolated anti-KLB antibody, wherein the antibody competes for binding to human KLB or monkey KLB with the anti-KLB antibody according to any one of claims 1 to 8.

10. The anti-KLB antibody according to any one of claims 1 to 9, wherein the anti-KLB antibody has one or more of the following characteristics:
A. the anti-KLB antibody has activation activity on cells expressing hKLB&hFGFR1c; preferably, the anti-KLB antibody results in an activation fold of greater than 10 for CHOK1-hKLB&hFGFR1c, and/or the anti-KLB antibody results in an activation fold of greater than 2 for HEK293T-hKLB&hFGFR1c cells;
B. the anti-KLB antibody has weak activation activity on recombinant cells expressing hFGFR1c; preferably, the anti-KLB antibody results in an activation fold of less than 1 for HEK293-hFGFR1c cells;
C. the anti-KLB antibody has weak activation activity on cells expressing hKLB&hFGFR2c, hKLB&hFGFR3c, and/or hKLB&hFGFR4; preferably, the anti-KLB antibody results in an activation fold of less than 2 for L6-hKLB&hFGFR2c and/or L6-hKLB&hFGFR4;
D. the anti-KLB antibody is capable of binding to both human KLB and monkey KLB; preferably, the anti-KLB antibody is capable of binding to human KLB and monkey KLB with an EC₅₀ value of less than 2.0E-9M, the EC₅₀ value being detected by flow cytometry; and
E. the anti-KLB antibody is capable of binding to human KLB; preferably, the antibody is capable of binding to human KLB with a KD value of less than 3.0E-9M, the KD value being detected by the Biacore method.

11. A pharmaceutical composition, comprising:
a therapeutically effective amount of the anti-KLB antibody according to any one of claims 1 to 10 and one or more pharmaceutically acceptable carriers, diluents, buffers, or excipients,
wherein preferably, the pharmaceutical composition further comprises at least one second therapeutic agent.

12. An isolated nucleic acid encoding the anti-KLB antibody according to any one of claims 1 to 10.

13. A host cell, comprising the nucleic acid according to claim 12.

14. A method for treating a disease, wherein the method comprises the step of administering to a subject in need thereof a therapeutically effective amount of the anti-KLB antibody according to any one of claims 1 to 10 or the pharmaceutical composition according to claim 11;
preferably, the disease is a disease associated with FGF21 and/or FGF19;
more preferably, the disease is selected from the group consisting of NASH, type 2 diabetes mellitus, obesity, dyslipidemia, cardiovascular diseases, and metabolic syndrome.

15. A method for inducing FGF21 and/or FGF19 signaling, wherein the method comprises contacting a cell expressing KLB and FGFR with the anti-KLB antibody according to any one of claims 1 to 10.
